(19) Europäisches Patentamt
European Patent Office
Office européen des brevets

(11) EP 1 267 948 B1

(12) **EUROPEAN PATENT SPECIFICATION**

(45) Date of publication and mention of the grant of the patent:
**08.12.2010 Bulletin 2010/49**

(21) Application number: **01904742.2**

(22) Date of filing: **09.02.2001**

(51) Int Cl.:
***A61L 2/00*** *(2006.01)*

(86) International application number:
**PCT/SE2001/000269**

(87) International publication number:
**WO 2001/045487 (28.06.2001 Gazette 2001/26)**

(54) **ANAL INCONTINENCE TREATMENT APPARATUS WITH WIRELESS ENERGY SUPPLY**

BEHANDLUNGSAPPARAT FÜR ANAL-INKONTINENZ MIT DRAHTLOSER ENERGIEVERSORGUNG

APPAREIL DE TRAITEMENT DE L'INCONTINENCE ANALE AVEC ALIMENTATION D'ENERGIE SANS FIL

(84) Designated Contracting States:
**AT BE CH CY DE DK ES FI FR GB GR IE IT LI LU MC NL PT SE TR**

(30) Priority: **10.02.2000 US 502092**
**10.02.2000 US 502073**

(43) Date of publication of application:
**02.01.2003 Bulletin 2003/01**

(73) Proprietor: **Abdomica AG**
**6340 Baar (CH)**

(72) Inventors:
- **FORSELL, Peter**
**CH-6313 Menzingen (CH)**
- **JAKOBSSON, Arne**
**F-06600 Antibes (FR)**

(74) Representative: **Rosenquist, Per Olof**
**Bergenstrahle & Lindvall AB**
**P.O. Box 17704**
**118 93 Stockholm (SE)**

(56) References cited:
**FR-A1- 2 387 642**
**FR-A1- 2 692 777**
**US-A- 5 509 888**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 267 948 B1

## Description

The present invention relates to an anal incontinence treatment apparatus for treatment of a patient, who suffers from anal incontinence, comprising a restriction device implantable in the patient for engaging the colon, rectum or anus to restrict a faecal passageway therein, wherein the restriction device is operable to change the restriction of the faecal passageway. The term "patient" includes an animal or a human being.

Anal incontinence is a widespread disease. Several kinds of sphincter plastic surgery are used today to remedy anal incontinence. There is a prior manually operated sphincter system in an initial clinical trial phase where a hydraulic sphincter system connected to an elastic reservoir (balloon) placed in the scrotum is developed. A disadvantage of this system is that thick, hard fibrosis is created around the reservoir by pump movements making the system useless sooner or later.

U.S. Pat. No. 5593443 discloses a hydraulic anal sphincter under both reflex and voluntary control. A pressure controlled inflatable artificial sphincter is disclosed in U.S. Pat. No. 4222377.

The closest prior art is considered to be French patent FR2692777.

The object of the present invention is to provide a new convenient anal incontinence treatment apparatus, the performance of which may be affected by the patient at any time after operation, in particular when need arise over the course of a day, so that the patient substantially always is satisfied or comfortable.

This object is obtained by an apparatus of the kind as claimed in appended claim 1.

As a result, the advantage is achieved that the anal incontinence disease treatment apparatus of the invention provides simple and effective energy transmission, which ensures long reliable function of the apparatus, possibly for the rest of the patient's life.

The restriction device preferably controls the cross-sectional area of the faecal passageway in the colon or rectum, which gives the advantage that the patient is enabled to adjust the cross-sectional area of the faecal passageway, i.e. to open and close the faecal passageway, whenever the patient likes during the day.

Generally, the apparatus comprises an energy transforming device implantable in the patient for transforming the energy wirelessly transmitted by the energy transmission device from a first form into a second form different than the first form.

The energy transforming device may comprise at least one semiconductor type of component or a circuitry of such semiconductor components. The semiconductor component may comprise a transistor or microchip or similar electronic components. However, the semiconductor component may not comprise rectifying diodes.

In accordance with a main embodiment of the invention, the energy transforming device comprises at least one element having a positive region and a negative region and adapted to create an energy field between the positive and negative regions when exposed to the energy of the first form transmitted by the energy transmission device, so that the energy field provides the energy of the second form. Typically, the above-mentioned semiconductor component may include such an element.

In accordance with a preferred embodiment of the invention, the element comprises an electrical junction element capable of inducing an electric field between the positive and negative regions when exposed to the energy of the first form transmitted by the energy transmission device, whereby the energy of the second form comprises electric energy.

Consequently, the restriction device suitably is electrically operated, whereby the positive and negative regions of the electrical junction element supply electric energy for the operation of the restriction device. The apparatus suitably comprises implantable electric conductors connected to the positive and negative regions of the electrical junction element, whereby the electrical junction element is capable of supplying an electric current, such as a direct current, a pulsating direct current, a combination of a direct and pulsating direct current, an alternating current or a combination of a direct and alternating current, via the conductors. Furthermore, the electrical junction element may be capable of supplying a frequency, amplitude, or frequency and amplitude modulated analog, digital, or a combination of analog and digital signal, which is used in connection with control of the restriction device.

The element, preferably in the form of an electrical semiconductor junction element, should be designed to generate an output current exceeding 1 $\mu$A when exposed to the energy of the first form transmitted by the energy transmission device. Suitably the electrical junction element forms a flat and thin sheet and has a volume of less than 2000 $cm^3$ to be suited for subcutaneous implantation, so that the electrical junction element can be located just behind the skin of the patient. Alternatively, it would be possible to implant the element in the thorax or cephalic region of the patient, or in an orifice of the patient's body and under the mucosa or intraluminar outside the mucosa of the orifice. Of course, all the components of the energy transforming device including the electrical junction element in contact with the patient's body should be of biocompatible material.

For in vitro appliances, a particular type of an electrical semiconductor junction element has been commonly used, namely a so-called p-n (positive/negative) junction element, typically in the form of solar cells. A solar cell transfers solar energy in the form of visible light into electric energy in the form of direct current. For example, a p-n junction element may comprise two layers of semiconductor, one p-type (positive) and the other n-type (negative), sandwiched together to form a "p-n junction". This p-n junction induces an electric field across the element when absorbing quanta of light (photons).

To be more precise, the quanta of light transfer their energy to some of the semiconductor's electrons, which are then able to move about through the material. For each such negatively charged electron, a corresponding positive charge - a "hole" - is created. In an ordinary semiconductor, these electrons and holes recombine after a short time and their energy is wasted as heat. However, when the electrons and holes are swept across the p-n junction in opposite directions by the action of the electric field, the separation of charge induces a voltage across the p-n junction element. By connecting the p-n junction element to an external circuit, the electrons are able to flow thereby creating a current.

Surprisingly, it has been proved that although both the skin and subcutis absorb energy from an external light beam directed against the skin portion behind which a properly designed p-n junction element is located, the light energy transmitted through the skin can induce a current from the p-n junction element strong enough (minimum 1 μA) to enable the operation of the electrically operated restriction device. Thus, such a p-n junction element is now for the first time used for in vivo applications.

The apparatus may comprise an implantable pulse generator for generating electrical pulses from the energy of the second form produced by the energy field.

Generally, the energy transforming device is adapted to transform the energy of the first form directly or indirectly into the energy of the second form.

In accordance with a preferred embodiment of the invention, the energy of the second form comprises electric energy and the energy transforming device comprises a capacitor, which may be adapted to produce electric pulses from the transformed electric energy. Preferably, the capacitor may be adapted to produce the pulses as the energy transforming device transforms the energy of the first form transmitted by the energy transmission device into the electric energy of the second form. The capacitor should be small to facilitate implantation thereof; i.e. its capacity may not be more than 0,1 μF.

The apparatus may comprise an implantable stabiliser for stabilising the energy of the second form. Where the energy of the second form comprises electric current the stabiliser may comprise at least one capacitor of the type described above.

The apparatus comprises implantable electrical components. Where the electrical components include a capacitor of the type described above or an accumulator, at least one, preferably a single, voltage level guard may advantageously be provided, wherein the charge and discharge of the capacitor or accumulator is controlled by use of the voltage level guard. As a result, there is no need for any implanted current detector and/or charge level detector for the control of the capacitor, which makes the apparatus simple and reliable.

In a particular embodiment of the invention, the wireless energy of the first form comprises sound waves and the energy of the second form comprises electric energy, wherein the energy transforming device is adapted to directly transform the sound waves into electric energy.

The apparatus comprises an implantable motor or pump for operating the restriction device, wherein the motor or pump is powered by the transformed energy.

In accordance with a main aspect of the invention, the energy transmission device may be adapted to transmit wireless energy for direct use in connection with the operation of the restriction device, as the wireless energy is being transmitted. The advantage of directly using energy as it is transmitted is that the apparatus can be of a very simple design and the few components involved makes the apparatus extremely reliable. For example, the energy transmission device may be adapted to directly power the motor or pump with wireless energy. The wireless energy may comprise a magnetic field or electromagnetic waves, suitably in the form of a signal, for direct power of the motor or pump. All the various functions of the motor and associated components described in the present specification may be used where applicable.

As an alternative to the above-noted main aspect of the invention, the energy transforming device may be adapted to supply the energy of the second form for direct use in connection with the operation of the restriction device, as the energy of the first form is being transformed into the energy of the second form. Consequently, the energy transforming device may be adapted to directly power the motor or pump with the energy of the second form.

Generally, the energy transforming device directly operates the restriction device with the energy of the second form in a non-magnetic, non-thermal or non-mechanical manner.

Where the apparatus comprises a motor, which may be adapted to directly or intermittently operate the restriction device, the energy transforming device may power the motor with the energy of the second form. Suitably, the restriction device is operable to perform a reversible function and the motor is capable of reversing said function.

In accordance with another embodiment of the invention, the restriction device comprises a hydraulic restriction device, and the apparatus comprises an implantable pump for operating the hydraulic restriction device, wherein the energy transforming device supplies the energy of the second form for driving the pump. Preferably, the pump is not a plunger type of pump, but may comprise a peristaltic or membrane pump.

The energy transforming device preferably is capable of generating as the energy of the second form a current exceeding 1 μA, when transferring the energy of the first form transmitted by the energy transmission device.

The apparatus may comprise an implantable adjustment device for adjusting the restriction device to change the restriction of the faecal passageway. In accordance with a first alternative the adjustment device is adapted to mechanically adjust the restriction device. In accordance with a second alternative the adjustment device is adapted to hydraulically adjust the restriction device by using implanted hydraulic means. Such hydraulic means may not use hydraulic fluid of the kind having a viscosity that substantially increases when exposed to heat or a magnetic field.

The apparatus of the present invention is not limited to the use of visible light for the wireless transmission of energy. Thus, in accordance with a broad aspect of the invention, the energy transmission device transmits energy by at least one wireless signal, preferably containing radiant energy.

The wireless signal may comprises a wave signal, for example an electromagnetic wave signal, such as an infrared light signal, a visible light signal, an ultra violet light signal, a laser signal, a micro wave signal, a radio wave signal, an x-ray radiation signal, and a gamma radiation signal. Where applicable, one or more of the above signals may be combined. Alternatively, the wave signal may comprise a sound wave signal, such as an ultrasonic signal. Generally, the wireless signal may comprise a digital, analog or a digital and analog signal.

The energy of the first form transmitted by the energy transmission device may comprise an electric or magnetic field transmitted in pulses, for example digital pulses. Furthermore, the energy transforming device may transform the energy of the first form, which may comprise polarised energy, into a direct current, pulsating direct current, a combination of a direct and pulsating direct current, an alternating current or a combination of a direct and alternating current. Alternatively, the energy of the first form may comprise kinetic energy.

The energy of the second form may comprise a frequency, amplitude or frequency and amplitude modulated analog, digital or combined analog and digital signal.

The restriction device may be non-inflatable, i.e. with no hydraulic fluid involved for the adjustments of the restriction device. This eliminates problems with fluid leaking from the restriction device.

The apparatus suitably comprises implantable electric conductors connected to the energy transforming device, whereby the energy transforming device is capable of supplying an electric current, such as direct current, a pulsating direct current, a combination of a direct and pulsating direct current, an alternating current or a combination of a direct and alternating current, via the conductors. Furthermore, the energy transforming device may be capable of supplying a frequency, amplitude, or frequency and amplitude modulated analog, digital, or a combination of analog and digital signal, which is used in connection with control of the restriction device.

The apparatus comprises an implantable operation device for operating the restriction device and a control device for controlling the operation device, wherein the energy transforming device powers the operation device with the energy of the second form. The operation device preferably comprises a motor, for example an electric linear motor or an electric rotary motor that is controlled by the control device to rotate a desired number of revolutions. Optionally, an implantable gearing may be connected to the motor. The electric motor may have electrically conductive parts made of plastics. Alternatively, the motor may comprise a hydraulic or pneumatic fluid motor, wherein the control device controls the fluid flow through the fluid motor. Motors currently available on the market are getting smaller and smaller. Furthermore, there is a great variety of control methods and miniaturised control equipment available. For example, the number of revolutions of a rotary motor may be analysed by a Hall-element just a few mm in size.

In accordance with another embodiment of the invention, the restriction device comprises hydraulic means and the operation device is adapted to conduct a hydraulic fluid in the hydraulic means. The operation device comprises a fluid conduit connected to the hydraulic means of the restriction device, and a reservoir for fluid, wherein the reservoir forms part of the conduit. The reservoir may form a fluid chamber with a variable volume, and the operation device may be adapted to distribute fluid from the chamber to the hydraulic means of the restriction device by reduction of the volume of the chamber and to withdraw fluid from the hydraulic means to the chamber by expansion of the volume of the chamber. The operation device suitably comprises an implantable motor used for reducing and expanding the volume of the chamber. Also, the operation device may comprise a pump for pumping the hydraulic fluid in the hydraulic means of the restriction device. All of the hydraulic components involved are preferably devoid of any non-return valve. This is of great advantage, because with valves involved there is always a risk of malfunction due to improperly working valves, especially when long time periods passes between valve operations.

The control device may be adapted to reverse the operation device by shifting polarity of the energy of the second form. Where the operation device comprises an electric motor the energy of the second form suitably comprises electric energy.

In accordance with yet another embodiment of the invention, the restriction device is operable to perform a reversible function, such as enlarging and restricting the faecal passageway, and there is a reversing device implanted in the patient for reversing the function performed by the restriction device. Such a reversing function preferably involves enlarging and restricting the faecal passageway by the restriction device, suitably in a stepless manner. In this connection, the control device suitably controls the reversing device, which may include a switch, to reverse the function performed by the restriction device. The reversing device may comprise hydraulic means including a valve for shifting the flow direction of a fluid in the hydraulic means. Alternatively, the reversing device may comprise a mechanical reversing device, such as a switch or a gearbox.

Where the reversing device comprises a switch it may be operable by the energy of the second form. In this case, the control device suitably controls the operation of the switch by shifting polarity of the energy of the second form supplied to the switch. The switch may comprise an electric switch and the source of energy may supply electric energy for the operation of the switch.

In accordance with an advantageous embodiment of the invention, the apparatus further comprises an energy storage device implanted in the patient for storing the energy of the second form and for supplying energy in connection with the operation of the restriction device. The implanted energy storage device preferably comprises an electric source of energy, such as an accumulator, a rechargeable battery or a combination of an accumulator and rechargeable battery.

The apparatus may further comprise a switch implantable in the patient for switching the operation of the restriction device and a source of energy implantable in the patient. This embodiment is particularly suited for applications where the energy transmission efficiency of the apparatus is insufficient, i.e. where the implanted restriction device is to perform more advanced operations. Such a source of energy preferably is a battery. Alternatively, the source of energy is an accumulator that also may store the energy of the second form.

In accordance with a first alternative, the switch is operated by the energy of the second form supplied by the energy storage device to switch from an off mode, in which the source of energy is not in use, to an on mode, in which the source of energy supplies energy for the operation of the restriction device. In this case, the implanted source of energy may comprise a battery, preferably having a lifetime of at least 10 years, or an accumulator. However, other kinds of sources are also conceivable, such as a nuclear source of energy or a chemical source of energy (fuel cells).

In accordance with a second alternative, the apparatus further comprises a remote control for controlling the supply of energy of the implanted source of energy, wherein the switch is operated by the energy of the second form supplied by the energy storage device to switch from an off mode, in which the remote control is prevented from controlling the source of energy and the source of energy is not in use, to a standby mode, in which the remote control is permitted to control the source of energy to supply energy for the operation of the restriction device.

In accordance with a third alternative, the energy storage device is omitted, wherein the switch is operated by the energy of the second form supplied by the energy transforming device to switch from an off mode, in which the remote control is prevented from controlling the source of energy and the source of energy is not in use, to a standby mode, in which the remote control is permitted to control the source of energy to supply energy for the operation of the restriction device.

In accordance with a fourth alternative, also the remote control is omitted, wherein the switch is operated by the energy of the second form supplied by the energy transforming device to switch from an off mode, in which the source of energy is not in use, to an on mode, in which the source of energy supplies energy for the operation of the restriction device. Where applicable, in the described embodiments the switch may switch when the energy transmission device is transmitting wireless energy, preferably while the transferred energy of the second form is stabilised by an implanted capacitor, which may temporarily (for a few seconds) store the energy of the second form.

In the above noted third and fourth alternatives, the energy transmission device may be substituted for the energy transforming device, whereby the switch is operated by the energy of the first form.

The switch mentioned above may comprise an electronic switch or, where applicable, a mechanical switch.

The advantage of using a switch above all is increased control safety; i.e. interfering signals in the patient's surroundings cannot affect the implanted restriction device. Furthermore, the lifetime of the implanted source of energy will be significantly prolonged, since the energy consumption of the apparatus will be reduced to a minimum. During the above-mentioned standby mode, the remote control uses energy from the implanted source of energy. By means of the energy transmission device energy may be transmitted to activate the switch to connect the implanted source of energy only when energy is required in connection with the operation of the restriction device.

All of the above embodiments may be combined with at least one implantable sensor for sensing at least one physical parameter of the patient, wherein the control device may control the restriction device in response to signals from the sensor. For example, the sensor may comprise a pressure sensor for directly or indirectly sensing the pressure against the restriction device, human tissue or in the faecal passageway, or the pressure against the colon or rectum. The control device may comprise an internal control unit implanted in the patient for, preferably directly, controlling the restriction device in response to signals from the sensor. In response to signals from the sensor, for example pressure, the patient's position or any other important physical parameter, the internal control unit may send information thereon to outside the patient's body. The control unit may also automatically control the restriction device in response to signals from the sensor. For example, the control unit may control the restriction device to further restrict or close the faecal passageway in response to the sensor sensing that the patient is lying, or enlarge the faecal passageway or produce an alarm signal in response to the sensor sensing an abnormally high pressure against the restriction device.

Alternatively, the control device may comprise an external control unit outside the patient's body for, suitably directly, controlling the restriction device in response to signals from the sensor. The external control unit may store information on the physical parameter sensed by the sensor and may be manually operated to control the restriction device based on the stored information. In addition, there may be at least one implantable sender for sending information on the physical parameter sensed by the sensor.

An external data communicator may be provided outside the patient's body and an internal data communicator may be implanted in the patient for communicating with the external communicator. The internal communicator may feed data related to the patient, or related to the restriction device, back to the external communicator. Alternatively or in combination, the external communicator may feed data to the internal communicator. The internal communicator may suitably feed data related to at least one physical signal of the patient.

The apparatus may further comprise an implantable programmable control unit for controlling the restriction device, preferably over time in accordance with an activity schedule program. This will advance the apparatus and make possible an adaptation of the apparatus to the individual patients.

Many of the above embodiments are suitably remote controlled. Thus, the apparatus advantageously comprises a wireless remote control transmitting at least one wireless control signal for controlling the restriction device. With such a remote control it will be possible to adapt the function of the apparatus to the patient's need in a daily basis, which is beneficial with respect to the treatment of the patient. The control signal may comprise a frequency, amplitude or frequency or amplitude modulated signal. Furthermore, the control signal may comprise an analog or a digital signal, or a combination of an analog and digital signal.

The wireless remote control may be capable of obtaining information on the condition of the implanted restriction device and of controlling the restriction device in response to the information. Also, The remote control may be capable of sending information related to the restriction device from inside the patient's body to the outside thereof.

In a particular embodiment of the invention, the wireless remote control comprises at least one external signal transmitter or transceiver and at least one internal signal receiver or transceiver implantable in the patient. In another particular embodiment of the invention, the wireless remote control comprises at least one external signal receiver or transceiver and at least one internal signal transmitter or transceiver implantable in the patient.

The wireless remote control may transmit a carrier signal for carrying the control signal, wherein the carrier signal is frequency, amplitude or frequency and amplitude modulated. The carrier signal may also comprise digital, analog or a combination of digital and analog signals. Such signals may comprise wave signals. Also the control signal used with the carrier signal may be frequency, amplitude or frequency and amplitude modulated, and be digital, analog or combined digital and analog.

The control signal may comprise a wave signal, for example, a sound wave signal, such as an ultrasound wave signal, an electromagnetic wave signal, such as an infrared light signal, a visible light signal, an ultra violet light signal, a laser signal, a micro wave signal, a radio wave signal, an x-ray radiation signal, or a gamma radiation signal. Where applicable, two or more of the above signals may be combined.

The control signal may be digital or analog, and may comprise an electric or magnetic field. Suitably, the wireless remote control may transmit an electromagnetic carrier wave signal for carrying the digital or analog control signal. For example, use of an analog carrier wave signal carrying a digital control signal would give safe communication. The control signal may be transmitted in pulses by the wireless remote control.

The energy transmission device may function different from or similar to the energy transforming device. For example, the energy transmission and transforming devices function differently when the energy transmission device comprises a coil used for transmitting the energy of the first form and the energy transforming device comprises an electrical junction element for transforming the transmitted energy into the energy of the second form. The energy transmission and transforming devices function similar to each other when the energy transmission device comprises a coil used for transmitting the energy of the first form and the energy transforming device also comprises a coil for transforming the transmitted energy into the energy of the second form.

In accordance with an alternative embodiment of the invention, the apparatus comprises an activatable source of energy implantable in the patient, wherein the source of energy is activated by wireless energy transmitted by the energy transmission device, to supply energy which is used in connection with the operation of the restriction device.

The implantable restriction device suitably is embedded in a soft or gel-like material. For example, a silicone material having hardness less than 20 Shore.

All the above described various components, such as the motor, pump and capacitor, may be combined in the different embodiments where applicable. Also the various functions described in connection with the above embodiments of the invention may be used in different applications, where applicable.

All the various ways of transferring, transforming and controlling energy presented in the present specification may be practised by using all of the various components and solutions described.

The present invention also provides an implanting method, comprising the steps of providing an anal incontinence disease treatment apparatus as described above, cutting an opening in a patient's mucosa in an orifice of the patient's body, and implanting the energy transforming device in the patient's body through the opening. Alternatively, the cutting step may comprise cutting an opening in the patient's skin and the implanting step may comprise implanting the energy transforming device in the patient's body through the opening.

There is also provided a laparascopical implanting method, in accordance with a first alternative, comprising the steps of providing an anal incontinence disease treatment apparatus as described above, placing at least two laparascopic cannula within a patient's body, and implanting the energy transforming device in the patient's body by using the at least two laparascopic cannula.

In accordance with a second alternative there is provided a laparoscopic surgical method of implanting an anal incontinence disease treatment apparatus as described above, comprising the steps of, a) placing at least two laparoscopic trocars within a patient's body, b) using at least one dissecting tool inserted through the laparoscopic trocar, dissecting the region of the colon, rectum or anus, c) introducing a restriction device of the apparatus through the trocars, and d) placing the restriction device in engagement with the colon, rectum or anus to create a restricted stoma. The method may further comprise implanting an energy transforming device of the apparatus, for example subcutaneously, in the abdomen, thorax or cephalic region, or other locations in the patient's body.

The invention is described in more detail in the following with reference to the accompanying drawings, in which

FIGURES 1 to 12 are schematic block diagrams illustrating twelve embodiments, respectively, of the anal incontinence disease treatment apparatus of the invention, in which wireless energy is transmitted from outside a patient's body to energy consuming components of the apparatus implanted in the patient.
FIGURE 13 is a schematic block diagram illustrating conceivable combinations of implanted components for achieving various communication options;
FIGURE 14 illustrates an electrical junction element for use in the apparatus of the present invention; and
FIGURE 15 illustrates the apparatus in accordance with the invention implanted in a patient;
FIGURE 16 is a block diagram illustrating remote control components of an embodiment of the invention, in which wireless energy is transmitted by the use of electromagnetic signals; and
FIGURE 17 is a schematic view of exemplary circuitry used for the components of the block diagram of FIGURE 16.

Referring to the drawing figures, like reference numerals designate identical or corresponding elements throughout the several figures.

FIGURE 1 schematically shows a most simple embodiment of the anal incontinence disease apparatus of the invention having some parts implanted in a patient and other parts located outside the patient's body. Thus, in FIGURE 1 all parts placed to the right of the patient's skin 2 are implanted and all parts placed to the left of the skin 2 are located outside the patient's body.

The apparatus of FIGURE 1 comprises an implanted operable restriction device 4, which engages the patient's colon (or alternatively engages the rectum) to form a restricted faecal passageway in the colon. The restriction device 4 is capable of performing a reversible function, i.e. to enlarge and reduce the cross-sectional area of the faecal passageway, whereby the restriction device 4 works as an artificial sphincter. An implanted energy transforming device 6 is adapted to supply energy consuming components of the restriction device 4 with energy via a power supply line 12. An external energy transmission device 10 includes a wireless remote control for transmitting a wireless signal, which is received by a signal receiver incorporated in the implanted energy transforming device 6. The implanted energy transforming device 6 transforms energy from the signal into electric energy that is supplied via the power supply line 12.

FIGURE 2 shows an embodiment of the invention identical to that of FIGURE 1, except that a reversing device in the form of an electric switch 14 energy also is implanted in the patient for reversing the restriction device 4. The wireless remote control of the external energy transmission device 10 transmits a wireless signal that carries energy and the implanted energy transforming device 6 transforms the wireless energy into a current for operating the switch 14. When the polarity of the current is shifted by the energy transforming device 6 the switch 14 reverses the function performed by the restriction device 4.

FIGURE 3 shows an embodiment of the invention identical to that of FIGURE 1, except that an operation device in the form of a motor 15 for operating the restriction device 4 also is implanted in the patient. The motor 15 is powered with energy from the energy transforming device 6, as the remote control of the external energy transmission device 10 transmits a wireless signal to the receiver of the energy transforming device 6.

FIGURE 4 shows an embodiment of the invention identical to that of FIGURE 1, except that an assembly 16 including a motor/pump unit 18 and a fluid reservoir 20 also is implanted in the patient. In this case the restriction device 4 is hydraulically operated, i.e. hydraulic fluid is pumped by the motor/pump unit 18 from the reservoir 20 through a conduit 22 to the restriction device 4 to reduce the cross-sectional area of the faecal passageway, and hydraulic fluid is pumped by the motor/pump unit 18 back from the restriction device 4 to the reservoir 20 to enlarge the cross-sectional area. The implanted energy transforming device unit 6 transforms wireless energy into a current, for example a current, for powering the motor/pump unit 18 via an electric power supply line 24.

FIGURE 5 shows an embodiment of the invention comprising the external energy transmission device 10 with its wireless remote control, the restriction device 4, in this case hydraulically operated, and the implanted energy transforming device 6, and further comprising an implanted hydraulic fluid reservoir 30, an implanted motor/pump unit 32 and an implanted reversing device in the form of a hydraulic valve shifting device 34. The motor of the motor/pump unit 32 is an electric motor. In response to a control signal from the wireless remote control of the external energy transmission device 10, the implanted energy transforming device 6 powers the motor/pump unit 32 with energy from the energy carried by the control signal, whereby the motor/pump unit 32 distributes hydraulic fluid between the reservoir 30 and the restriction device 4. The remote control of the energy transmission device 10 controls the shifting device 34 to shift the hydraulic fluid flow direction between one direction in which the fluid is pumped by the motor/pump unit 32 from the reservoir 30 to the restriction device 4 to reduce the cross-sectional area of the faecal passageway, and another opposite direction in which the fluid is pumped by the motor/pump unit 32 back from the restriction device 4 to the reservoir 30 to enlarge the cross-sectional area.

FIGURE 6 shows an embodiment of the invention identical to that of FIGURE 1, except that a control unit 36 controlled by the wireless remote control of the external energy transmission device 10, an accumulator 38 and a capacitor 40 also are implanted in the patient. The control unit 36 stores electric energy received from the energy transforming device 6 in the accumulator 38, which supplies energy to the restriction device 4. In response to a control signal from the wireless remote control of the energy transmission device 10, the control unit 6 either releases electric energy from the accumulator 38 and transfers the released energy via power lines 42 and 44, or directly transfers electric energy from the energy transforming device 6 via a power line 46, the capacitor 40, which stabilises the electric current, a power line 48 and the power line 44, for the operation of the restriction device 4.

In accordance with one alternative, the capacitor 40 in the embodiment of FIGURE 6 may be omitted. In accordance with another alternative, the accumulator 38 in this embodiment may be omitted.

FIGURE 7 shows an embodiment of the invention identical to that of FIGURE 1, except that a battery 50 for supplying energy for the operation of the restriction device 4 and an electric switch 52 for switching the operation of the restriction device 4 also are implanted in the patient. The switch 52 is operated by the energy supplied by the energy transforming device 6 to switch from an off mode, in which the battery 50 is not in use, to an on mode, in which the battery 50 supplies energy for the operation of the restriction device 4.

FIGURE 8 shows an embodiment of the invention identical to that of FIGURE 7, except that a control unit 36 controllable by the wireless remote control of the external energy transmission device 10 also is implanted in the patient. In this case, the switch 52 is operated by the energy supplied by the energy transforming device 6 to switch from an off mode, in which the wireless remote control is prevented from controlling the control unit 36 and the battery is not in use, to a standby mode, in which the remote control is permitted to control the control unit 36 to release electric energy from the battery 50 for the operation of the restriction device 4.

FIGURE 9 shows an embodiment of the invention identical to that of FIGURE 8, except that an accumulator 38 is substituted for the battery 50 and the implanted components are interconnected differently. In this case, the accumulator 38 stores energy from the energy transforming device 6. In response to a control signal from the wireless remote control of the external energy transmission device 10, the implanted control unit 36 controls the switch 52 to switch from an off mode, in which the accumulator 38 is not in use, to an on mode, in which the accumulator 38 supplies energy for the operation of the restriction device 4.

FIGURE 10 shows an embodiment of the invention identical to that of FIGURE 9, except that a battery 50 also is implanted in the patient and the implanted components are interconnected differently. In response to a control signal from the wireless remote control of the external energy transmission device 10, the implanted control unit 36 controls the accumulator 38 to deliver energy for operating the switch 52 to switch from an off mode, in which the battery 50 is not in use, to an on mode, in which the battery 50 supplies electric energy for the operation of the restriction device 4.

Alternatively, the switch 52 may be operated by energy supplied by the accumulator 38 to switch from an off mode, in which the wireless remote control is prevented from controlling the battery 50 to supply electric energy and is not in use, to a standby mode, in which the wireless remote control is permitted to control the battery 50 to supply electric energy for the operation of the restriction device 4.

FIGURE 11 shows an embodiment of the invention identical to that of FIGURE 7, except that a motor 15, a mechanical reversing device in the form of a gearbox 54 and a control unit 36 for controlling the gearbox 54 also are implanted in the patient. The implanted control unit 36 controls the gearbox 54 to reverse the function performed by the restriction device 4 (mechanically operated).

FIGURE 12 shows an embodiment of the invention identical to that of FIGURE 10 except that the implanted components are interconnected differently. Thus, in this case the battery 50 powers the control unit 36 when the accumulator 38, suitably a capacitor, activates the switch 52 to switch to an on mode. When the switch 52 is in its on mode the control unit 36 is permitted to control the battery 50 to supply, or not supply, energy for the operation of the restriction device 4.

FIGURE 13 schematically shows conceivable combinations of implanted components of the apparatus for achieving various communication options. Basically, there are the implanted restriction device 4, control unit 36 and motor/pump unit 18, and the external energy transmission device 10 including the external wireless remote control. As already described above the wireless remote control transmits a control signal which is received by the implanted control unit 36, which in turn controls the various implanted components of the apparatus.

A sensor 56 may be implanted in the patient for sensing a physical parameter of the patient, such as the pressure in the faecal passageway. The implanted control unit 36, or alternatively the external wireless remote control of the energy transmission device 10, may control the restriction device 4 in response to signals from the sensor 56. A transceiver may be combined with the sensor 56 for sending information on the sensed physical parameter to the external wireless remote control. The wireless remote control may comprise a signal transmitter or transceiver and the implanted control unit 36 may comprise a signal receiver or transceiver. Alternatively, the wireless remote control may comprise a signal receiver or transceiver and the implanted control unit 36 may comprise a signal transmitter or transceiver. The above transceivers, transmitters and receivers may be used for sending information or data related to the restriction device 4 from inside the patient's body to the outside thereof.

Where the motor/pump unit 18 and battery 50 for powering the motor/pump unit 18 are implanted, the battery 50 may be equipped with a transceiver for sending information on the condition of the battery 50.

Those skilled in the art will realise that the above various embodiments according to FIGURES 1-13 could be combined in many different ways. For example, the energy operated switch 14 could be incorporated in any of the embodiments of FIGURES 3,6-12, the hydraulic shifting device 34 could be incorporated in the embodiment of FIGURE 4, and the gearbox 54 could be incorporated in the embodiment of FIGURE 3.

FIGURE 14 shows an energy transforming device in the form of an electrical junction element 58 for use in any of the above embodiments according to FIGURES 1-13. The element 58 is a flat p-n junction element comprising a p-type semiconductor layer 60 and an n-type semiconductor layer 62 sandwiched together. A light bulb 64 is electrically connected to opposite sides of the element 58 to illustrate how the generated current is obtained. The output of current from such a p-n junction element 58 is correlated to the temperature. See the formula below.

$$I = I0\ (\exp(qV/kT)-1)$$

where

I is the external current flow,
I0 is the reverse saturation current,
q is the fundamental electronic charge of 1.602 x 10-19 coulombs,
V is the applied voltage,
k is the Boltzmann constant, and
T is the absolute temperature.

Under large negative applied voltage (reverse bias), the exponential term becomes negligible compared to 1.0, and I is approximately -I0. 10 is strongly dependent on the temperature of the junction and hence on the intrinsic-carrier concentration. I0 is larger for materials with smaller bandgaps than for those with larger bandgaps. The rectifier action of the diode - that is, its restriction of current flow to only one direction - is in this particular embodiment the key to the operation of the p-n junction element 58.

An alternative way to design a p-n junction element is to deposit a thin layer of semiconductor onto a supporting material which does not absorb the kind of energy utilised in the respective embodiments. For use with wirelessly transmitted energy in terms of light waves, glass could be a suitable material. Various materials may be used in the semiconductor layers such as but not limited to cadmium telluride, copper-indium-diselenide and silicon. It is also possible to use a multilayer structure with several layers of p and n-type materials to improve efficiency.

The electric energy generated by the p-n junction element 58 could be of the same type as generated by solar cells, in which the negative and positive fields create a direct current. Alternatively, the negative and positive semiconductor layers may change polarity following the transmitted waves, thereby generating an alternating current.

The p-n junction element 58 is designed to make it suited for implantation. Thus, all the external surfaces of the element 58 in contact with the human body are made of a biocompatible material. The p-n junction semiconductors are designed to operate optimally at a body temperature of 37° C because the current output, which should be more than 1 μA, is significantly depending on temperature as shown above. Since both the skin and subcutis absorb energy, the relation between the sensitivity or working area of the element 58 and the intensity or strength of the wireless energy transmission is considered. The p-n junction element 58 preferably is designed flat and small. Alternatively, if the element 58 is made in larger sizes it should be flexible, in order to adapt to the patient's body movements. The volume of the element 58 should be kept less than 2000 $cm^3$.

FIGURE 15 generally illustrates how any of the above-described embodiments of the anal incontinence disease treatment apparatus of the invention may be implanted in a patient. Thus, a restriction device 4 implanted in a patient engages the colon 66 to form an artificial sphincter around the faecal passageway in the colon 66. An implanted operation device 68, such as an electric motor or a motor/pump assembly, operates the restriction device 4 through a transmission member 70, such as a mechanical transmission cord or a fluid tube. An energy transforming device in the form of an element 6 having a positive region and a negative region, as described above in more detail, is placed underneath the skin of the patient.

Wireless energy carried by a signal transmitted by a wireless remote control of an external energy transmission device 10 at least partly penetrates the patient's skin and hits the element 6. The energy thus hitting the element 6 is transformed into energy of a different form that is suited for powering the operation device 68. For example, where the operation device 68 is an electric motor the element 6 comprises an electric p-n junction element that transforms the wireless energy into an electric current for powering the electric motor. Where the operation device 68 comprises a pump, the element 6 may transform the wireless energy into kinetic energy for powering the pump.

The transformed energy may be utilised for directly operating the restriction device 4 or, where the restriction device 4 is electrically operated, for storage in a capacitor and/or an accumulator for later or parallel use. Preferably (but not necessarily) the element 6 is controlled by a microprocessor. The wireless remote control of the external energy transmission device 10 is used to control the utilisation of the transmitted energy and any function or command to/from the implanted restriction device 4.

FIGURE 16 shows the basic parts of a wireless remote control of the apparatus of the invention including an electric motor 128 for operating a restriction member, for example of the type illustrated in FIGURE 15. In this case, the remote control is based on the transmission of electromagnetic wave signals, often of high frequencies in the order of 100 kHz - 1 gHz, through the skin 130 of the patient. In FIGURE 15, all parts placed to the left of the skin 130 are located outside the patient's body and all parts placed to the right of the skin 130 are implanted. Any suitable remote control system may be used.

An external signal transmitting antenna 132 is to be positioned close to a signal receiving antenna 134 implanted close to the skin 130. As an alternative, the receiving antenna 134 may be placed for example inside the abdomen of the patient. The receiving antenna 134 comprises a coil, approximately 1-100 mm, preferably 25 mm in diameter, wound with a very thin wire and tuned with a capacitor to a specific high frequency. A small coil is chosen if it is to be implanted under the skin of the patient and a large coil is chosen if it is to be implanted in the abdomen of the patient. The transmitting antenna 132 comprises a coil having about the same size as the coil of the receiving antenna 134 but wound with a thick wire that can handle the larger currents that is necessary. The coil of the transmitting antenna 132 is tuned to the same specific high frequency as the coil of the receiving antenna 134.

An external control unit 136 comprises a microprocessor, a high frequency electromagnetic wave signal generator and a power amplifier. The microprocessor of the control unit 136 is adapted to switch the generator on/off and to modulate signals generated by the generator to send digital information via the power amplifier and the antennas 132,134 to an implanted control unit 138. To avoid that accidental random high frequency fields trigger control commands, digital signal codes are used. A conventional keypad placed on the external control unit 136 is connected to the microprocessor thereof. The keypad is used to order the microprocessor to send digital signals to either contract or enlarge the restriction device. The microprocessor starts a command by applying a high frequency signal on the antenna 132. After a short time, when the signal has energised the implanted parts of the control system, commands are sent to contract or enlarge the restriction device in predefined steps. The commands are sent as digital packets in the form illustrated below.

| | | | |
|---|---|---|---|
| Start pattern, 8 bits | Command, 8 bits | Count, 8 bits | Checksum, 8 bits |

The commands are sent continuously during a rather long time period (e.g. about 30 seconds or more). When a new contract or enlarge step is desired the Count byte is increased by one to allow the implanted control unit 138 to decode and understand that another step is demanded by the external control unit 136. If any part of the digital packet is erroneous, its content is simply ignored.

Through a line 140, an implanted energiser unit 126 draws energy from the high frequency electromagnetic wave signals received by the receiving antenna 134. The energiser unit 126 stores the energy in an energy storage device, such as a large capacitor, powers the control unit 138 and powers the electric motor 128 via a line 142.

The control unit 138 comprises a demodulator and a microprocessor. The demodulator demodulates digital signals sent from the external control unit 136. The microprocessor of the control unit 138 receives the digital packet, decodes it and, provided that the power supply of the energiser unit 126 has sufficient energy stored, sends a signal via a signal line 144 to the motor 128 to either contract or enlarge the restriction device depending on the received command code.

Alternatively, the energy stored in the energy storage device of the energiser unit may only be used for powering a switch, and the energy for powering the motor 128 may be obtained from another implanted energy source of relatively high capacity, for example a battery. In this case the switch is adapted to connect said battery to the control unit 138 in an on mode when said switch is powered by the energy storage device and to keep the battery disconnected from the control unit in a standby mode when the switch is not powered.

With reference to FIGURE 17, the remote control schematically described above will now be described in accordance with a more detailed embodiment. The external control unit 136 comprises a microprocessor 146, a signal generator 148 and a power amplifier 150 connected thereto. The microprocessor 146 is adapted to switch the signal generator 148 on/off and to modulate signals generated by the signal generator 148 with digital commands that are sent to implanted components of the apparatus. The power amplifier 150 amplifies the signals and sends them to the external signal transmitting antenna 132. The antenna 132 is connected in parallel with a capacitor 152 to form a resonant circuit tuned to the frequency generated by the signal generator 148.

The implanted signal receiving antenna coil 134 forms together with a capacitor 154 a resonant circuit that is tuned to the same frequency as the transmitting antenna 132. The signal receiving antenna coil 134 induces a current from the received high frequency electromagnetic waves and a rectifying diode 160 rectifies the induced current, which charges a storage capacitor 158. A coil 156 connected between the antenna coil 134 and the diode 160 prevents the capacitor 158 and the diode 160 from loading the circuit of the signal receiving antenna 134 at higher frequencies. Thus, the coil 156 makes it possible to charge the capacitor 158 and to transmit digital information using amplitude modulation.

A capacitor 162 and a resistor 164 connected in parallel and a diode 166 forms a detector used to detect amplitude modulated digital information. A filter circuit is formed by a resistor 168 connected in series with a resistor 170 connected in series with a capacitor 172 connected in series with the resistor 168 via ground, and a capacitor 174, one terminal of which is connected between the resistors 168,170 and the other terminal of which is connected between the diode 166 and the circuit formed by the capacitor 162 and resistor 164. The filter circuit is used to filter out undesired low and high frequencies. The detected and filtered signals are fed to an implanted microprocessor 176 that decodes the digital information and controls the motor 128 via an H-bridge 178 comprising transistors 180,182,184 and 186. The motor 128 can be driven in two opposite directions by the H-bridge 178.

The microprocessor 176 also monitors the amount of stored energy in the storage capacitor 158. Before sending signals to activate the motor 128, the microprocessor 176 checks whether the energy stored in the storage capacitor 158 is enough. If the stored energy is not enough to perform the requested operation, the microprocessor 176 waits for the received signals to charge the storage capacitor 158 before activating the motor 128.

## Claims

1. An anal incontinence disease treatment apparatus for treatment of a patient, who suffers from anal incontinence, comprising
a restriction device (4) implantable in the patient for engaging the colon, rectum or anus to form a restricted faecal passageway therein, the restriction device being operable to perform a reversible function involving enlarging and restricting the faecal passageway,
an operation device (15, 18, 32, 128; 18, 32) implantable in the patient for operating the restriction device,
an energy transmission device (10) for wireless transmission of energy from outside the patient's body to inside the patient's body for use in connection with the operation of the restriction device including powering the operation device, and
an energy transforming device (6) implantable in the patient for transforming the energy of the first form wirelessly transmitted by the energy transmission device into energy of a second form different than the energy of the first form, the restriction device being operable in response to the energy of the second form to change the restriction of the faecal passageway, when the restriction device is implanted, wherein the operation device comprises:

    (i) a motor (15, 18, 32, 128) implantable in the patient, wherein the energy of the second form is used for powering the motor to reversibly operate the restriction device (4); or

    a pump (18, 32) implantable in the patient, wherein the energy of the second form is used for powering the pump to operate the restriction device (4), **characterized in that** the apparatus further comprises at least one implantable voltage level guard.

2. An apparatus according to claim 1, wherein the energy transforming device comprises at least one element having a positive region and a negative region, the element is capable of creating an energy field between the positive and negative regions when exposed to the energy of the first form transmitted by the energy transmission device, and the energy field produces the energy of the second form.

**3.** An apparatus according to claim 2, wherein the element comprises an electrical junction element, and the electrical junction element is capable of inducing an electric field between the positive and negative regions when exposed to the energy of the first form transmitted by the energy transmission device, whereby the energy of the second form comprises electric energy.

**4.** An apparatus according to claim 3, wherein the restriction device is electrically operated, and the positive and negative regions of the electrical junction element supply electric energy for the operation of the restriction device.

**5.** An apparatus according to claim 4, further comprising electric conductors connected to the positive and negative regions of the electrical junction element, whereby the electrical junction element is capable of supplying an electric current via the conductors.

**6.** An apparatus according to claim 5, wherein the electrical junction element is capable of supplying a direct current or pulsating direct current via the conductors.

**7.** An apparatus according to claim 5, wherein the electrical junction element is capable of supplying an alternating current or a combination of a direct and alternating current via the conductors.

**8.** An apparatus according to claim 4, wherein the electrical junction element is capable of supplying a frequency or amplitude modulated signal.

**9.** An apparatus according to claim 4, wherein the electrical junction element is capable of supplying an analog or digital signal.

**10.** An apparatus according to any of claims 1-9, wherein the energy transforming device forms a flat and thin sheet, and has a volume of less than 2000 $cm^3$.

**11.** An apparatus according to claim 1, wherein the energy transforming device is adapted to transform the energy of the first form directly or indirectly into the energy of the second form.

**12.** An apparatus according to claim 1, wherein the energy transforming device is adapted to directly power the motor or pump by the transformed energy, as the energy of the second form is being transformed from the energy of the first form.

**13.** An apparatus according to any of claims 11-12, wherein the wireless energy of the first form comprises sound waves and the energy of the second form comprises electric energy.

**14.** An apparatus according to any of claims 1-13, wherein the energy transforming device comprises a capacitor and the energy of the second form comprises electric energy.

**15.** An apparatus according to claim 14, wherein the capacitor is adapted to produce electric pulses from the transformed electric energy.

**16.** An apparatus according to claim 15, wherein the capacitor is adapted to produce the pulses of the electric energy, as the energy transforming device transforms the energy of the first form transmitted by the energy transmission device into the electric energy of the second form.

**17.** An apparatus according to claim 1, further comprising an implantable stabiliser for stabilising the energy of the second form.

**18.** An apparatus according to claim 17, wherein the energy of the second form comprises electric current and the stabiliser comprises at least one capacitor.

**19.** An apparatus according to claim 1, further comprising implantable electrical components including a capacitor or accumulator.

**20.** An apparatus according to any of claims 1-19, further comprising implantable electrical components including a single voltage level guard.

**21.** An apparatus according to claim 19 or 20, wherein the electrical components are devoid of any current detector and/or charge level detector.

**22.** An apparatus according to any of claims 19-21, further comprising an implantable capacitor or accumulator, wherein the charge or discharge of the capacitor or accumulator is controlled by use of the voltage level guard.

**23.** An apparatus according to any of claims 14-16, 18 and 22, wherein the capacitor has a capacity less than 0,1 $\mu$F.

**24.** An apparatus according to claim 1, wherein the energy transmission device is adapted to transmit wireless energy for direct use in connection with the operation of the restriction device, as the wireless energy is being transmitted.

**25.** An apparatus according to claim 24, wherein the energy transmission device is adapted to directly power the motor or pump with wireless energy.

**26.** An apparatus according to claim 25, wherein the energy transmission device is adapted to transmit wireless energy in the form of a magnetic field or electromagnetic waves for direct power of the motor or pump.

**27.** An apparatus according to claim 1, wherein the energy transforming device is adapted to supply the energy of the second form for direct use in connection with the operation of the restriction device, as the energy of the first form is being transformed into the energy of the second form.

**28.** An apparatus according to claim 27, wherein the energy transforming device is adapted to directly power the motor or pump with the energy of the second form.

**29.** An apparatus according to claim 28, wherein the energy transforming device directly operates the restriction device with the energy of the second form in a non-magnetic, non-thermal or non-mechanical manner.

**30.** An apparatus according to any of claims 1, wherein the motor directly or intermittently operates the restriction device, and the energy transforming device powers the motor with the energy of the second form.

**31.** An apparatus according to any of claims 1-24 and 27, wherein the restriction device comprises a hydraulic restriction device, and further comprising an implantable pump for operating the hydraulic restriction device, the energy transforming device supplying the energy of the second form for driving the pump.

**32.** An apparatus according to any of claims 25,26,28 and 31, wherein the pump is not a plunger type of pump.

**33.** An apparatus according to any of the preceding claims, wherein the energy transforming device is capable of generating as the energy of the second form a current exceeding 1 $\mu$A, when transferring the energy of the first form transmitted by the energy transmission device.

**34.** An apparatus according to any of the preceding claims, further comprising an adjustment device for adjusting the restriction device to change the restriction of the faecal passageway, wherein the adjustment device is adapted to mechanically adjust the restriction device, or adapted to hydraulically adjust the restriction device by using hydraulic means which is devoid of hydraulic fluid of the kind having a viscosity that substantially increases when exposed to heat or a magnetic field.

**35.** An apparatus according to any of claims 1-10, wherein the energy transforming device comprises at least one semiconductor type of component.

**36.** An apparatus according to claim 35, wherein the energy transforming device comprises a circuitry of semiconductor components.

**37.** An apparatus according to claim 35, wherein the semiconductor component comprises a transistor or microchip or similar electronic components excluding rectifying diodes.

**38.** An apparatus according to claim 36 or 37, wherein the semiconductor component comprises at least one element having a positive region and a negative region, the element is capable of creating an energy field between the positive and negative regions when exposed to the energy of the first form transmitted by the energy transmission device, and the energy field produces the energy of the second form.

**39.** An apparatus according to claim 1, further comprising a control device for controlling the motor.

**40.** An apparatus according to claim 29 or 39, further comprising an implantable gearing connected to the motor.

**41.** An apparatus according to claims 39 and 40, wherein the motor comprises a rotary motor and the control device controls the rotary motor to rotate a desired number of revolutions.

**42.** An apparatus according to claim 39, wherein the motor comprises a linear motor.

**43.** An apparatus according to claim 39 and 40, wherein the motor comprises a hydraulic or pneumatic fluid motor, and the control device controls the fluid motor.

**44.** An apparatus according to claim 39, wherein the motor comprises an electric motor having electrically conductive parts made of plastics.

**45.** An apparatus according to claim 39, wherein the restriction device comprises hydraulic means.

**46.** An apparatus according to claim 45, further comprising a fluid conduit connected to the hydraulic means of the restriction device, and a reservoir for fluid, the reservoir forming part of the conduit.

**47.** An apparatus according to claim 46, wherein the hydraulic means and conduit are devoid of any non-return valve.

**48.** An apparatus according to claim 47, wherein the reservoir forms a fluid chamber with a variable volume, and the motor is adapted to distribute fluid from the chamber to the hydraulic means of the restriction device by reduction of the volume of the chamber and to withdraw fluid from the hydraulic means to the chamber by expansion of the volume of the chamber.

**49.** An apparatus according to claim 48, wherein the operation device comprises an implantable motor used for reducing and expanding the volume of the chamber.

**50.** An apparatus according to any of claims 45-48, wherein the pump is adapted to pump the hydraulic fluid in the hydraulic means of the restriction device.

**51.** An apparatus according to claim 39, wherein the control device shifts polarity of the energy of the second form to reverse the motor.

**52.** An apparatus according to claim 39 or 49, wherein the motor comprises an electric motor and the energy of the second form comprises electric energy.

**53.** An apparatus according to claim 52, further comprising a reversing device implantable in the patient for reversing the function performed by the restriction device.

**54.** An apparatus according to claims 39 and 53, wherein the control device controls the reversing device to reverse the function performed by the restriction device.

**55.** An apparatus according to claim 53 or 54, wherein the reversing device comprises hydraulic means including a valve for shifting the flow direction of a fluid flow in the hydraulic means.

**56.** An apparatus according to claim 53 or 54, wherein the reversing device comprises a mechanical reversing device.

**57.** An apparatus according to claim 56, wherein the reversing device comprises a gearbox.

**58.** An apparatus according to claim 53 or 54, wherein the reversing device comprises a switch.

**59.** An apparatus according to claim 58, wherein the switch is operable by the energy of the second form.

**60.** An apparatus according to claim 59, wherein the control device controls the operation of the switch by shifting polarity of the energy of the second form.

**61.** An apparatus according to claim 58 or 59, wherein the switch comprises an electric switch and the energy of the second form comprises electric energy.

**62.** An apparatus according to any one of claims 15,16 and 39, wherein the control device is adapted to control the energy transforming device to produce the energy of the second form in a train of energy pulses for direct use in connection with the operation of the restriction device.

**63.** An apparatus according to claim 1, further comprising an energy storage device implantable in the patient for storing the energy of the second form and for supplying energy in connection with the operation of the restriction device.

**64.** An apparatus according to claim 63, wherein the energy storage device comprises an accumulator.

**65.** An apparatus according to claim 64, wherein the energy of the second form comprises electric energy and the energy storage device comprises an electric accumulator.

**66.** An apparatus according to claim 65, wherein the electric accumulator comprises at least one capacitor or at least one rechargeable battery, or a combination of at least one capacitor and at least one rechargeable battery.

**67.** An apparatus according to any of claims 1,58,63-66, further comprising a switch implantable in the patient for directly or indirectly switching the operation of the restriction device.

**68.** An apparatus according to claim 67, further comprising a source of energy implantable in the patient, wherein the switch is operated by the energy of the second form supplied by the energy storage device to switch from an off mode, in which the source of energy is not in use, to an on mode, in which the source of energy supplies energy for the operation of the restriction device.

**69.** An apparatus according to claim 67, further comprising a source of energy implantable in the patient, and a remote control for controlling the supply of energy of the source of energy, wherein the switch is operated by the energy of the second form supplied by the energy storage device to switch from an off mode, in which the remote control is prevented from controlling the source of energy and the source of energy is not in use, to a standby mode, in which the remote control is permitted to control the source of energy to supply energy for the operation of the restriction device.

**70.** An apparatus according to claim 67, further comprising a source of energy implantable in the patient for supplying energy for the operation of the restriction device, wherein the switch is operated by the energy of the second form supplied by the energy transforming device to switch from an off mode, in which the source of energy is not in use, to an on mode, in which the source of energy supplies energy for the operation of the restriction device.

**71.** An apparatus according to claim 67, further comprising a source of energy implantable in the patient for supplying energy for the operation of the restriction device, and a remote control for controlling the supply of energy of the implantable source of energy, wherein the switch is operated by the energy of the second form supplied by the energy transforming device to switch from an off mode, in which the remote control is prevented from controlling the source of energy and the source of energy is not in use, to a standby mode, in which the remote control is permitted to control the source of energy to supply energy for the operation of the restriction device.

**72.** An apparatus according to claim 67, further comprising a source of energy implantable in the patient for supplying energy for the operation of the restriction device, wherein the switch is operated by the energy of the first form supplied by the energy transmission device to switch from an off mode, in which the source of energy is not in use, to an on mode, in which the source of energy supplies energy for the operation of the restriction device.

**73.** An apparatus according to claim 67, further comprising a source of energy implantable in the patient for supplying energy for the operation of the restriction device, and a remote control for controlling the supply of energy of the implantable source of energy, wherein the switch is operated by the energy of the first form supplied by the energy transmission device to switch from an off mode, in which the remote control is prevented from controlling the source of energy and the source of energy is not in use, to a standby mode, in which the remote control is permitted to control the source of energy to supply energy for the operation of the restriction device.

**74.** An apparatus according to claim 1, wherein the restriction device is electrically operated, and the energy of the second form comprises electric energy.

**75.** An apparatus according to claim 74, further comprising electric conductors connected to the energy transforming device, whereby the energy transforming device is capable of supplying an electric current via the conductors.

**76.** An apparatus according to claim 1, wherein the energy transforming device is capable of supplying a frequency, amplitude or frequency and amplitude modulated signal.

**77.** An apparatus according to claim 1, wherein the energy transforming device is capable of supplying an analog, digital or a combination of an analog and digital signal.

**78.** An apparatus according to claim 1, further comprising an activatable source of energy implantable in the patient, wherein the source of energy is activated by wireless energy transmitted by the energy transmission device, to supply energy which is used in connection with the operation of the restriction device.

**79.** An apparatus according to claim 1, wherein the energy transmission device transmits energy by at least one wireless signal.

**80.** An apparatus according to claim 79, wherein the signal contains radiant energy.

**81.** An apparatus according to claim 80, wherein the signal comprises a wave signal.

**82.** An apparatus according to claim 81, wherein the wave signal comprises an electromagnetic wave signal including one of an infrared light signal, a visible light signal, an ultra violet light signal, a laser signal, a micro wave signal, a radio wave signal, an x-ray radiation signal, and a gamma radiation signal.

**83.** An apparatus according to claim 82, wherein the wave signal comprises a sound or ultrasound wave signal.

**84.** An apparatus according to any one of claims 79-83, wherein the signal comprises a digital or analog signal, or a combination of a digital and analog signal.

**85.** An apparatus according to claim 1, wherein the energy of the first form transmitted by the energy transmission device comprises an electric, an electromagnetic or a magnetic field, or a combination thereof.

**86.** An apparatus according to claim 85, wherein the electric, electromagnetic or magnetic field, or the combination thereof is transmitted in pulses or digital pulses, or a combination of pulses and digital pulses by the energy transmission device.

**87.** An apparatus according to claim 1, wherein the energy of a first form transmitted by the energy transmission device comprises an electric, an electromagnetic or a magnetic field, or a combination thereof.

**88.** An apparatus according to claim 87, wherein the electric, electromagnetic or magnetic field, or the combination thereof is transmitted in waves or analog pulses or a combination thereof by the energy transmission device.

**89.** An apparatus according to any one of claims 1-88, wherein the energy transmitted by the energy transmission device comprises polarised energy.

**90.** An apparatus according to claim 1, wherein the energy transforming device transforms the energy of the first form into a direct current or pulsating direct current, or a combination of a direct current and pulsating direct current.

**91.** An apparatus according to claim 1, wherein the energy transforming device transforms the energy of the first form into an alternating current or a combination of a direct and alternating current.

**92.** An apparatus according to claim 1, further comprising an implantable pulse generator for generating electrical pulses from the energy of the second form produced by the energy field.

**93.** An apparatus according to any one of the preceding claims, further comprising at least one implantable sensor for sensing at least one physical parameter of the patient.

**94.** An apparatus according to claim 93, wherein the sensor comprises a pressure sensor for directly or indirectly sensing as the physical parameter the pressure in the faecal passageway or the pressure against the colon, rectum or anus.

**95.** An apparatus according to claim 94, further comprising a control device for controlling the restriction device in response to signals from the sensor.

**96.** An apparatus according to claim 95, wherein the control device comprises an internal control unit implantable in the patient for controlling the restriction device in response to signals from the sensor.

**97.** An apparatus according to claim 96, wherein the internal control unit directly controls the restriction device in response to signals from the sensor.

**98.** An apparatus according to claim 97, wherein the control device comprises an external control unit outside the patient's body for controlling the restriction device in response to signals from the sensor.

**99.** An apparatus according to claim 98, wherein the external control unit stores information on the physical parameter sensed by the sensor and is manually operated to control the restriction device based on the stored information.

**100.** An apparatus according to any one of claims 93-99, further comprising at least one implantable sender for sending information on the physical parameter sensed by the sensor.

**101.** An apparatus according to any one of the preceding claims, further comprising a wireless remote control for transmitting at least one wireless control signal for controlling the restriction device.

**102.** An apparatus according to claim 101, wherein the control signal comprises a frequency, amplitude or frequency or amplitude modulated signal.

**103.** An apparatus according to claim 102, wherein the control signal comprises an analog or a digital signal, or a combination of an analog and digital signal.

**104.** An apparatus according to any of claims 101-103, wherein the remote control is capable of obtaining information on the condition of the implantable restriction device and to control the restriction device in response to the information.

**105.** An apparatus according to any of claims 101-104, wherein the remote control comprises an implantable control unit for controlling the restriction device.

**106.** An apparatus according to claim 105, wherein the control unit comprises a microprocessor.

**107.** An apparatus according to any one of claims 101-106, wherein the wireless remote control comprises at least one external signal transmitter or transceiver and at least one internal signal receiver or transceiver implantable in the patient.

**108.** An apparatus according to any one of claims 100-106, wherein the wireless remote control comprises at least one external signal receiver or transceiver and at least one internal signal transmitter or transceiver implantable in the patient.

**109.** An apparatus according to any one of claims 101-108, wherein the remote control is capable of sending information related to the restriction device from inside the patients body to the outside thereof.

**110.** An apparatus according to claim 109, wherein the remote control controls the restriction device in response to the information.

**111.** An apparatus according to any one of claims 101-110, wherein the remote control comprises a control signal transmitter for transmitting the wireless control signal, and the energy transmission device comprises the control signal transmitter, whereby energy is transmitted by the control signal.

**112.** An apparatus according to any one of claims 101-110, wherein the energy transmission device transmits energy by at least one signal separate from the control signal.

**113.** An apparatus according to any one of claims 101-110, wherein the remote control transmits a carrier signal for carrying the control signal.

**114.** An apparatus according to any one of claims 101-110, wherein the energy transmission device transmits energy by at least one signal, which is used as a carrier signal for the control signal transmitted by the remote control.

**115.** An apparatus according to claim 114, wherein the carrier signal is frequency, amplitude or frequency and amplitude modulated.

**116.** An apparatus according to claim 114 or 115, wherein the carrier signal comprises digital, analog or a combination of digital and analog signals.

**117.** An apparatus according to claim 116, wherein the signals comprise wave signals.

**118.** An apparatus according to any one of claims 101-117, wherein the control signal comprises a wave signal comprising one of a sound wave signal, an ultrasound wave signal, an electromagnetic wave signal, an infrared light signal, a visible light signal, an ultra violet light signal, a laser light signal, a micro wave signal, a radio wave signal, an x-ray radiation signal and a gamma radiation signal.

**119.** An apparatus according to any one of claims 101-117.
wherein the control signal comprises an electric or magnetic field, or a combined electric and magnetic field.

**120.** An apparatus according to claim 103, wherein the remote control transmits an electromagnetic carrier wave signal for carrying the digital or analog control signal.

**121.** An apparatus according to claim 1, wherein the energy of the second form used for operating the restriction device is wirelessly transmitted by the energy transforming device.

**122.** An apparatus according to claim 1, further comprising an implantable control unit for controlling the restriction device.

**123.** An apparatus according to claim 120, wherein the control unit is programmable for controlling the restriction device in accordance with a program.

**124.** An apparatus according to claim 123, wherein the control unit controls the restriction device over time in accordance with an activity schedule program

**125.** An apparatus according to any one of claims 122-124, further comprising an external wireless remote control for programming the implantable control unit.

**126.** An apparatus according to claim 1, further comprising an external data communicator and an implantable internal data communicator communicating with the external data communicator, wherein the internal communicator feeds data related to the restriction device back to the external data communicator or the external data communicator feeds data to the internal data communicator.

**127.** An apparatus according to claim 126, wherein the internal data communicator feeds data related to at least one physical signal of the patient.

**128.** An apparatus according to any one of the preceding claims, wherein the restriction device is adapted to control the restriction of the faecal passageway when implanted.

**129.** An apparatus according to any one of the preceding claims, wherein the restriction device is non-inflatable.

**130.** An apparatus according to claim 1, wherein one of the energy of the first form and the energy of the second form comprises magnetic energy, kinetic energy, sound energy, chemical energy, radiant energy, electromagnetic energy, photo energy, nuclear energy or thermal energy.

**131.** An apparatus according to claim 1, wherein one of the energy of the first form and the energy of the second form is non-magnetic, non-kinetic, non-chemical, non-sonic, non-nuclear or non-thermal.

**132.** An apparatus according to claim 1, wherein the energy transmission device functions different from the energy transforming device.

**133.** An apparatus according to claim 1, wherein the energy transmission device functions similar to the energy transforming device.

**134.** An apparatus according to any one of the preceding claims, wherein the energy transforming device is designed to be implanted subcutaneously or in the abdomen, thorax or cephalic region of the patient.

**135.** An apparatus according to any one of claims 1-134, wherein the energy transforming device is designed to be implanted in an orifice of the patient's body and under the mucosa or intraluminar outside the mucosa of the orifice.

**136.** An apparatus according to any of the preceding claims, wherein the restriction device is embedded in a soft or gel-like material.

**137.** An apparatus according to any of the preceding claims, wherein the restriction device is embedded in a silicone material having hardness less than 20 Shore.

## Patentansprüche

**1.** Behandlungsapparat für Anal-Inkontinenz eines Patienten, der an Anal-Inkontinenz leidet, umfassend
eine Beschränkungsvorrichtung (4), die im Patienten implantierbar ist, um in den Dickdarm, Mastdarm oder Anus einzugreifen, um darin einen beschränkten Fäkal-Durchgang zu bilden, wobei die Beschränkungsvornchtung betrieben werden kann, eine reversible Funktion auszuführen, die das Vergrößern und Beschränken des Fäkal-Durchgangs mit sich bringt,
eine Betriebsvorrichtung (15, 18, 32, 128; 18, 32), die im Patienten zum Betreiben der Beschränkungsvorrichtung implantierbar ist,
eine Energieübertragungsvorrichtung (10) zur drahtlosen Übertragung von Energie von außerhalb des Patientenkörpers zum Inneren des Patientenkörpers zur Verwendung in Verbindung mit dem Betrieb der Beschränkungsvorrichtung, umfassend das Antreiben der Betriebsvorrichtung, und
eine Energieumwandlungsvorrichtung (6), die im Patienten zum Umwandeln der Energie der ersten Form, die drahtlos von der Energieübertragungsvorrichtung übertragen wird, in Energie einer zweiten Form, die sich von der Energie der ersten Form unterscheidet, implantierbar ist, wobei die Beschränkungsvorrichtung als Reaktion auf die Energie der zweiten Form betrieben werden kann, um die Beschränkung des Fäkal-Durchgangs zu ändern, wenn die Beschränkungsvorrichtung implantiert wird, wobei die Betriebsvorrichtung umfasst:

(i) einen Motor (15, 18, 32, 128), der im Patienten implantierbar ist, wobei die Energie der zweiten Form zum Antreiben des Motors verwendet wird, um die Beschränkungsvorrichtung (4) umkehrbar zu betreiben; oder

eine Pumpe (18, 32), die im Patienten implantierbar ist, wobei die Energie der zweiten Form verwendet wird, um die Pumpe anzutreiben, um die Beschränkungsvorrichtung (4) zu betreiben, **dadurch gekennzeichnet, dass** der Apparat überdies mindestens eine implantierbare Spannungspegel-Schutzvorrichtung umfasst.

**2.** Apparat nach Anspruch 1, wobei die Energieumwandlungsvorrichtung mindestens ein Element umfasst, das einen positiven Bereich und einen negativen Bereich aufweist, das Element in der Lage ist, ein Energiefeld zwischen dem positiven und negativen Bereich zu erzeugen, wenn es der Energie der ersten Form ausgesetzt ist, die von der Energieübertragungsvorrichtung übertragen wird, und das Energiefeld die Energie der zweiten Form hervorruft.

**3.** Apparat nach Anspruch 2, wobei das Element ein elektrisches Verbindungselement umfasst, und das elektrische Verbindungselement in der Lage ist, ein elektrisches Feld zwischen dem positiven und negativen Bereich zu induzieren, wenn es der Energie der ersten Form, die von der Energieübertragungsvorrichtung übertragen wird, ausgesetzt ist, wobei die Energie der zweiten Form elektrische Energie umfasst.

**4.** Apparat nach Anspruch 3, wobei die Beschränkungsvorrichtung elektrisch betrieben wird und der positive und negative Bereich des elektrischen Verbindungselements elektrische Energie für den Betrieb der Beschränkungsvorrichtung zuführen.

**5.** Apparat nach Anspruch 4, überdies umfassend elektrische Leiter, die an den positiven und negativen Bereich des elektrischen Verbindungselements angeschlossen sind, wobei das elektrische Verbindungselement in der Lage ist, einen elektrischen Strom über die Leiter zuzuführen.

**6.** Apparat nach Anspruch 5, wobei das elektrische Verbindungselement in der Lage ist, einen Gleichstrom oder einen pulsierenden Gleichstrom über die Leiter zuzuführen.

**7.** Apparat nach Anspruch 5, wobei das elektrische Verbindungselement in der Lage ist, einen Wechselstrom oder eine Kombination aus Gleich- und Wechselstrom über die Leiter zuzuführen.

**8.** Apparat nach Anspruch 4, wobei das elektrische Verbindungselement in der Lage ist, ein frequenz- oder amplitudenmoduliertes Signal zuzuführen.

**9.** Apparat nach Anspruch 4, wobei das elektrische Verbindungselement in der Lage ist, ein analoges oder digitales Signal zuzuführen.

**10.** Apparat nach einem der Ansprüche 1-9, wobei die Energieumwandlungsvorrichtung ein flaches und dünnes Blech bildet und ein Volumen von weniger als 2000 $cm^3$ aufweist.

**11.** Apparat nach Anspruch 1, wobei die Energieumwandlungsvorrichtung angepasst ist, die Energie der ersten Form direkt oder indirekt in die Energie der zweiten Form umzuwandeln.

**12.** Apparat nach Anspruch 1, wobei die Energieumwandlungsvorrichtung angepasst ist, den Motor oder die Pumpe durch die umgewandelte Energie direkt anzutreiben, wenn die Energie der zweiten Form von der Energie der ersten Form umgewandelt wird.

**13.** Apparat nach einem der Ansprüche 11-12, wobei die drahtlose Energie der ersten Form Schallwellen umfasst und die Energie der zweiten Form elektrische Energie umfasst.

**14.** Apparat nach einem der Ansprüche 1-13, wobei die Energieumwandlungsvorrichtung einen Kondensator umfasst und die Energie der zweiten Form elektrische Energie umfasst.

**15.** Apparat nach Anspruch 14, wobei der Kondensator angepasst ist, elektrische Impulse von der umgewandelten elektrischen Energie hervorzurufen.

**16.** Apparat nach Anspruch 15, wobei der Kondensator angepasst ist, die Impulse der elektrischen Energie hervorzurufen, wenn die Energieumwandlungsvorrichtung die Energie der ersten Form, die von der Energieübertragungsvorrichtung übertragen wird, in die elektrische Energie der zweiten Form umwandelt.

**17.** Apparat nach Anspruch 1, überdies umfassend einen implantierbaren Stabilisator zum Stabilisieren der Energie der zweiten Form.

**18.** Apparat nach Anspruch 17, wobei die Energie der zweiten Form elektrischen Strom umfasst und der Stabilisator mindestens einen Kondensator umfasst.

**19.** Apparat nach Anspruch 1, überdies umfassend implantierbare elektrische Bauteile, umfassend einen Kondensator oder Akkumulator.

**20.** Apparat nach einem der Ansprüche 1-19, überdies umfassend implantierbare elektrische Bauteile, umfassend eine einzelne Spannungspegel-Schutzvorrichtung.

**21.** Apparat nach Anspruch 19 oder 20, wobei die elektrischen Bauteile frei von irgendeinem Stromdetektor und/oder Ladungspegeldetektor sind.

**22.** Apparat nach einem der Ansprüche 19-21, überdies umfassend einen implantierbaren Kondensator oder Akkumulator, wobei das Laden oder Entladen des Kondensators oder Akkumulators durch die Verwendung der Spannungspegel-Schutzvorrichtung gesteuert wird.

**23.** Apparat nach einem der Ansprüche 14-16, 18 und 22, wobei der Kondensator eine Kapazität von weniger als 0,1 μF aufweist.

**24.** Apparat nach Anspruch 1, wobei die Energieübertragungsvorrichtung angepasst ist, drahtlose Energie zur direkten Verwendung in Verbindung mit dem Betrieb der Beschränkungsvorrichtung zu übertragen, wenn die drahtlose Energie übertragen wird.

**25.** Apparat nach Anspruch 24, wobei die Energieübertragungsvorrichtung angepasst ist, den Motor oder die Pumpe direkt mit drahtloser Energie anzutreiben.

**26.** Apparat nach Anspruch 25, wobei die Energieübertragungsvorrichtung angepasst ist, drahtlose Energie in Form eines magnetischen Felds oder elektromagnetischer Wellen zu übertragen, um den Motor oder die Pumpe direkt anzutreiben.

**27.** Apparat nach Anspruch 1, wobei die Energieumwandlungsvorrichtung angepasst ist, die Energie der zweiten Form zur direkten Verwendung in Verbindung mit dem Betrieb der Beschränkungsvorrichtung zuzuführen, wenn die Energie der ersten Form in die Energie der zweiten Form umgewandelt wird.

**28.** Apparat nach Anspruch 27, wobei die Energieumwandlungsvorrichtung angepasst ist, den Motor oder die Pumpe direkt mit der Energie der zweiten Form anzutreiben.

**29.** Apparat nach Anspruch 28, wobei die Energieumwandlungsvorrichtung die Beschränkungsvorrichtung mit der Energie der zweiten Form in einer nicht magnetischen, nicht thermischen oder nicht mechanischen Weise direkt betreibt.

**30.** Apparat nach einem der Ansprüche 1, wobei der Motor die Beschränkungsvorrichtung direkt oder periodisch betreibt und die Energieumwandlungsvorrichtung den Motor mit der Energie der zweiten Form antreibt.

**31.** Apparat nach einem der Ansprüche 1-24 und 27, wobei die Beschränkungsvorrichtung eine hydraulische Beschränkungsvorrichtung umfasst und überdies eine implantierbare Pumpe zum Betreiben der hydraulischen Beschränkungsvorrichtung umfasst, wobei die Energieumwandlungsvorrichtung die Energie der zweiten Form zum Antreiben der Pumpe zuführt.

**32.** Apparat nach einem der Ansprüche 25, 26, 28 und 31, wobei die Pumpe keine Tauchkolbenpumpe ist.

**33.** Apparat nach einem der vorhergehenden Ansprüche, wobei die Energieumwandlungsvorrichtung in der Lage ist, als Energie der zweiten Form einen Strom zu erzeugen, der 1 μA übersteigt, wenn die Energie der ersten Form, die von der Energieübertragungsvorrichtung übertragen wird, übermittelt wird.

**34.** Apparat nach einem der vorhergehenden Ansprüche, überdies umfassend eine Anpassungsvorrichtung zum Anpassen der Beschränkungsvorrichtung, um die Beschränkung des Fäkal-Durchgangs zu ändern, wobei die Anpassungsvorrichtung angepasst ist, die Beschränkungsvorrichtung mechanisch anzupassen, oder angepasst ist, die Beschränkungsvorrichtung durch Verwendung eines Hydraulikmittels hydraulisch anzupassen, das frei von Hydraulikfluid der Art ist, die eine Viskosität aufweist, die im Wesentlichen zunimmt, wenn sie Wärme oder einem magnetischen Feld ausgesetzt ist.

**35.** Apparat nach einem der Ansprüche 1-10, wobei die Energieumwandlungsvorrichtung mindestens ein Halbleitertyp-Bauteil umfasst.

**36.** Apparat nach Anspruch 35, wobei die Energieumwandlungsvorrichtung eine Schaltungsanordnung aus Halbleiterbauteilen umfasst.

**37.** Apparat nach Anspruch 35, wobei die Halbleiterkomponente einen Transistor oder Mikrochip oder ähnlich elektronische Bauteile außer Gleichrichterdioden umfasst.

**38.** Apparat nach Anspruch 36 oder 37, wobei die Halbleiterkomponente mindestens ein Element umfasst, das einen positiven Bereich und einen negativen Bereich aufweist, wobei das Element in der Lage ist, ein Energiefeld zwischen dem positiven und negativen Bereich zu erzeugen, wenn es der Energie der ersten Form ausgesetzt ist, die von der Energieübertragungsvorrichtung übertragen wird und das Energiefeld die Energie der zweiten Form hervorruft.

**39.** Apparat nach Anspruch 1, überdies umfassend eine Steuervorrichtung zum Steuern des Motors.

**40.** Apparat nach Anspruch 29 oder 39, überdies umfassend ein implantierbares Getriebe, das an den Motor angeschlossen ist.

**41.** Apparat nach Ansprüchen 39 und 40, wobei der Motor einen Drehmotor umfasst und die Steuervorrichtung den Drehmotor steuert, um eine gewünschte Anzahl Umdrehungen zu drehen.

**42.** Apparat nach Anspruch 39, wobei der Motor einen Linearmotor umfasst.

**43.** Apparat nach Anspruch 39 und 40, wobei der Motor einen Hydraulik- oder Pneumatikfluidmotor umfasst und die Steuervorrichtung den Fluidmotor steuert.

**44.** Apparat nach Anspruch 39, wobei der Motor einen Elektromotor umfasst, der elektrisch leitende Teile aus Kunststoff aufweist.

**45.** Apparat nach Anspruch 39, wobei die Beschränkungsvorrichtung Hydraulikmittel umfasst.

**46.** Apparat nach Anspruch 45, überdies umfassend einen Fluidkanal, der an das Hydraulikmittel der Beschränkungsvorrichtung angeschlossen ist, und ein Reservoir für Fluid, wobei das Reservoir einen Teil des Kanals bildet.

**47.** Apparat nach Anspruch 46, wobei das Hydraulikmittel und der Kanal frei von irgendeinem Rückschlagventil sind.

**48.** Apparat nach Anspruch 47, wobei das Reservoir eine Fluidkammer mit einem veränderbaren Volumen bildet und der Motor angepasst ist, Fluid von der Kammer zu dem Hydraulikmittel der Beschränkungsvorrichtung durch Verminderung des Volumens der Kammer zu verteilen und Fluid von dem Hydraulikmittel zu der Kammer durch Ausdehnen des Volumens der Kammer abzuziehen.

**49.** Apparat nach Anspruch 48, wobei die Betriebsvorrichtung einen implantierbaren Motor umfasst, der zum Vermindern und Ausdehnen des Volumens der Kammer verwendet wird.

**50.** Apparat nach einem der Ansprüche 45-48, wobei die Pumpe angepasst ist, das Hydraulikfluid in das Hydraulikmittel der Beschränkungsvorrichtung zu pumpen.

**51.** Apparat nach Anspruch 39, wobei die Steuervorrichtung die Polarität der Energie der zweiten Form verschiebt, um den Motor im Umkehrbetrieb laufen zu lassen.

**52.** Apparat nach Anspruch 39 oder 49, wobei der Motor einen Elektromotor umfasst und die Energie der zweiten Form elektrische Energie umfasst.

**53.** Apparat nach Anspruch 52, überdies umfassend eine Umkehrvorrichtung, die im Patienten implantierbar ist, um die Funktion umzukehren, die von der Beschränkungsvorrichtung ausgeführt wird.

**54.** Apparat nach Ansprüchen 39 und 53, wobei die Steuerungsvorrichtung die Umkehrvorrichtung steuert, um die von der Beschränkungsvorrichtung ausgeführte Funktion umzukehren.

**55.** Apparat nach Anspruch 53 oder 54, wobei die Umkehrvorrichtung Hydraulikmittel umfasst, umfassend ein Ventil zum Verschieben der Flussrichtung eines Fluidflusses in dem Hydraulikmittel.

**56.** Apparat nach Anspruch 53 oder 54, wobei die Umkehrvorrichtung eine mechanische Umkehrvorrichtung umfasst.

**57.** Apparat nach Anspruch 56, wobei die Umkehrvorrichtung ein Getriebe umfasst.

**58.** Apparat nach Anspruch 53 oder 54, wobei die Umkehrvorrichtung einen Schalter umfasst.

**59.** Apparat nach Anspruch 58, wobei der Schalter durch die Energie der zweiten Form betrieben werden kann.

**60.** Apparat nach Anspruch 59, wobei die Steuervorrichtung den Betrieb des Schalters durch Verschieben der Polarität der Energie der zweiten Form steuert.

**61.** Apparat nach Anspruch 58 oder 59, wobei der Schalter einen elektrischen Schalter umfasst und die Energie der zweiten Form elektrische Energie umfasst.

**62.** Apparat nach einem der Ansprüche 15, 16 und 39, wobei die Steuervorrichtung angepasst ist, die Energieumwandlungsvorrichtung zu steuern, um die Energie der zweiten Form in einen Zug von Energieimpulsen zur direkten Verwendung in Verbindung mit dem Betrieb der Beschränkungsvorrichtung hervorzurufen.

**63.** Apparat nach Anspruch 1, überdies umfassend eine Energiespeichervorrichtung, die im Patienten zum Speichern der Energie der zweiten Form und zum Zuführen von Energie in Verbindung mit dem Betrieb der Beschränkungsvorrichtung implantierbar ist.

**64.** Apparat nach Anspruch 63, wobei die Energiespeichervorrichtung einen Akkumulator umfasst.

**65.** Apparat nach Anspruch 64, wobei die Energie der zweiten Form elektrische Energie umfasst und die Energiespeichervorrichtung einen elektrischen Akkumulator umfasst.

**66.** Apparat nach Anspruch 65, wobei der elektrische Akkumulator mindestens einen Kondensator oder mindestens eine wiederaufladbare Batterie oder eine Kombination aus mindestens einem Kondensator und mindestens einer wiederaufladbaren Batterie umfasst.

**67.** Apparat nach einem der Ansprüche 1, 58, 63-66, überdies umfassend einen Schalter, der im Patienten zum direkten oder indirekten Schalten des Betriebs der Beschränkungsvorrichtung implantierbar ist.

**68.** Apparat nach Anspruch 67, überdies umfassend eine Energiequelle, die im Patienten implantierbar ist, wobei der Schalter von der Energie der zweiten Form betrieben wird, die von der Energiespeichervorrichtung zugeführt wird, um von einer Aus-Betriebsart, in der die Energiequelle nicht in Verwendung ist, in eine Ein-Betriebsart zu schalten, in der die Energiequelle Energie für den Betrieb der Beschränkungsvorrichtung zuführt.

**69.** Apparat nach Anspruch 67, überdies umfassend eine Energiequelle, die im Patienten implantierbar ist, und eine Fernsteuerung zum Steuern des Zuführens der Energie der Energiequelle, wobei der Schalter durch die Energie der zweiten Form betrieben wird, die von der Energiespeichervorrichtung zugeführt wird, um von einer Aus-Betriebsart, in der verhindert wird, dass die Fernsteuerung die Energiequelle steuert und die Energiequelle nicht in Verwendung ist, in eine Bereitschafts-Betriebsart, in der erlaubt ist, dass die Fernsteuerung die Energiequelle steuert, zu schalten, um Energie für den Betrieb der Beschränkungsvorrichtung zuzuführen.

**70.** Apparat nach Anspruch 67, überdies umfassend eine Energiequelle, die im Patienten implantierbar ist, um Energie für den Betrieb der Beschränkungsvorrichtung zuzuführen, wobei der Schalter von der Energie der zweiten Form betrieben wird, die von der Energieumwandlungsvorrichtung zugeführt wird, um von einer Aus-Betriebsart, in der die Energiequelle nicht in Verwendung ist, in eine Ein-Betriebsart, in der die Energiequelle Energie für den Betrieb der Beschränkungsvorrichtung zuführt, zu schalten.

**71.** Apparat nach Anspruch 67, überdies umfassend eine Energiequelle, die im Patienten implantierbar ist, um Energie für den Betrieb der Beschränkungsvorrichtung zuzuführen, und eine Fernsteuerung zum Steuern der Energieversorgung der implantierbaren Energiequelle, wobei der Schalter von der Energie der zweiten Form betrieben wird, die von der Energieumwandlungsvorrichtung zugeführt wird, um von einer Aus-Betriebsart, in der verhindert wird, dass die Fernsteuerung die Energiequelle steuert und die Energiequelle nicht in Verwendung ist, in eine Bereitschafts-Betriebsart, in der erlaubt ist, dass die Fernsteuerung die Energiequelle steuert, zu schalten, um Energie für den Betrieb der Beschränkungsvorrichtung zuzuführen.

**72.** Apparat nach Anspruch 67, überdies umfassend eine Energiequelle, die im Patienten implantierbar ist, um Energie für den Betrieb der Beschränkungsvorrichtung zuzuführen, wobei der Schalter von der Energie der ersten Form betrieben wird, die von der Energieübertragungsvorrichtung zugeführt wird, um von einer Aus-Betriebsart, in der die Energiequelle nicht in Verwendung ist, in eine Ein-Betriebsart zu schalten, in der die Energiequelle Energie für den Betrieb der Beschränkungsvorrichtung zuführt.

**73.** Apparat nach Anspruch 67, überdies umfassend eine Energiequelle, die im Patienten implantierbar ist, um Energie für den Betrieb der Beschränkungsvorrichtung zuzuführen, und eine Fernsteuerung zum Steuern der Energiezufuhr der implantierbaren Energiequelle, wobei der Schalter von der Energie der ersten Form betrieben wird, die von der Energieübertragungsvorrichtung zugeführt wird, um von einer Aus-Betriebsart, in der verhindert wird, dass die Fernsteuerung die Energiequelle steuert und die Energiequelle nicht in Verwendung ist, in eine Bereitschafts-Betriebsart, in der erlaubt ist, dass die Fernsteuerung die Energiequelle steuert, zu schalten, um Energie für den Betrieb der Beschränkungsvorrichtung zuzuführen.

**74.** Apparat nach Anspruch 1, wobei die Beschränkungsvorrichtung elektrisch betrieben wird und die Energie der zweiten Form elektrische Energie umfasst.

**75.** Apparat nach Anspruch 74, überdies umfassend elektrische Leiter, die an die Energieumwandlungsvorrichtung angeschlossen sind, wobei die Energieumwandlungsvorrichtung in der Lage ist, einen elektrischen Strom über die Leiter zuzuführen.

**76.** Apparat nach Anspruch 1, wobei die Energieumwandlungsvorrichtung in der Lage ist, ein frequenz-, amplituden- oder frequenz- und amplitudenmoduliertes Signal zuzuführen.

**77.** Apparat nach Anspruch 1, wobei die Energieumwandlungsvorrichtung in der Lage ist, ein analoges, digitales oder eine Kombination aus einem analogen und digitalen Signal zuzuführen.

**78.** Apparat nach Anspruch 1, überdies umfassend eine aktivierbare Energiequelle, die im Patienten implantierbar ist, wobei die Energiequelle durch drahtlose Energie aktiviert wird, die von der Energieübertragungsvorrichtung übertragen wird, um Energie zuzuführen, die in Verbindung mit dem Betrieb der Beschränkungsvorrichtung verwendet wird.

**79.** Apparat nach Anspruch 1, wobei die Energieübertragungsvorrichtung Energie durch mindestens ein drahtloses Signal überträgt.

**80.** Apparat nach Anspruch 79, wobei das Signal Strahlungsenergie umfasst.

**81.** Apparat nach Anspruch 80, wobei das Signal ein Wellensignal umfasst.

**82.** Apparat nach Anspruch 81, wobei das Wellensignal ein elektromagnetisches Wellensignal, umfassend eines, ein Infrarot-Lichtsignal, ein sichtbares Lichtsignal, ein Ultraviolett-Lichtsignal, ein Lasersignal, ein Mikrowellensignal, ein Funkwellensignal, ein Röntgenstrahlen-Strahlungssignal oder ein GammaStrahlungssignal umfasst.

**83.** Apparat nach Anspruch 82, wobei das Wellensignal ein Schall- oder Ultraschall-Wellensignal umfasst.

**84.** Apparat nach einem der Ansprüche 79-83, wobei das Signal ein digitales und analoges Signal oder eine Kombination aus einem digitalen und analogen Signal umfasst.

**85.** Apparat nach Anspruch 1, wobei die Energie der ersten Form, die von der Energieübertragungsvorrichtung übertragen wird, ein elektrisches, ein elektromagnetisches oder ein magnetisches Feld oder eine Kombination davon umfasst.

**86.** Apparat nach Anspruch 85, wobei das elektrische, elektromagnetische oder magnetische Feld oder die Kombination davon in Impulsen oder Digitalimpulsen oder einer Kombination aus Impulsen und Digitalimpulsen von der Energieübertragungsvorrichtung übertragen wird.

**87.** Apparat nach Anspruch 1, wobei die Energie einer ersten Form, die von der Energieübertragungsvorrichtung übertragen wird, ein elektrisches, ein elektromagnetisches oder ein magnetisches Feld oder eine Kombination davon umfasst.

**88.** Apparat nach Anspruch 87, wobei das elektrische, elektromagnetische oder magnetische Feld oder die Kombination davon in Wellen oder Analogimpulsen oder einer Kombination davon von der Energieübertragungsvorrichtung übertragen wird.

**89.** Apparat nach einem der Ansprüche 1-88, wobei die Energie, die von der Energieübertragungsvorrichtung übertragen wird, polarisierte Energie umfasst.

**90.** Apparat nach Anspruch 1, wobei die Energieumwandlungsvorrichtung die Energie der ersten Form in einen Gleichstrom oder einen pulsierenden Gleichstrom oder eine Kombination aus einem Gleichstrom und pulsierenden Gleichstrom umwandelt.

**91.** Apparat nach Anspruch 1, wobei die Energieumwandlungsvorrichtung die Energie der ersten Form in einen Wechselstrom oder eine Kombination aus einem Gleich- und Wechselstrom umwandelt.

**92.** Apparat nach Anspruch 1, überdies umfassend einen implantierbaren Impulsgenerator zum Erzeugen elektrischer Impulse von der Energie der zweiten Form, die durch das Energiefeld hervorgerufen wird.

**93.** Apparat nach einem der vorhergehenden Ansprüche, überdies umfassend mindestens einen implantierbaren Sensor zum Erfassen mindestens eines physikalischen Parameters des Patienten.

**94.** Apparat nach Anspruch 93, wobei der Sensor einen Drucksensor umfasst, um als physikalischen Parameter den Druck in dem Fäkal-Durchgang oder den Druck gegen den Dickdarm, Mastdarm oder Anus zu erfassen.

**95.** Apparat nach Anspruch 94, überdies umfassend eine Steuervorrichtung zum Steuern der Beschränkungsvorrichtung in Reaktion auf Signale von dem Sensor.

**96.** Apparat nach Anspruch 95, wobei die Steuervorrichtung eine interne Steuereinheit umfasst, die im Patienten implantierbar ist, um die Beschränkungsvorrichtung in Reaktion auf Signale von dem Sensor zu steuern.

**97.** Apparat nach Anspruch 96, wobei die interne Steuereinheit die Beschränkungsvorrichtung in Reaktion auf Signale von dem Sensor direkt steuert.

**98.** Apparat nach Anspruch 97, wobei die Steuervorrichtung eine externe Steuereinheit außerhalb des Patientenkörpers zum Steuern der Beschränkungsvorrichtung in Reaktion auf Signale von dem Sensor umfasst.

**99.** Apparat nach Anspruch 98, wobei die externe Steuereinheit Informationen über den physikalischen Parameter speichert, der von dem Sensor erfasst wird, und manuell betätigt wird, um die Beschränkungsvorrichtung basierend auf den gespeicherten Informationen zu steuern.

**100.** Apparat nach einem der Ansprüche 93-99, überdies umfassend mindestens einen implantierbaren Sender zum Senden von Informationen über den physikalischen Parameter, der von dem Sensor erfasst wird.

**101.** Apparat nach einem der vorhergehenden Ansprüche, überdies umfassend eine drahtlose Fernsteuerung zum Übertragen mindestens eines drahtlosen Steuersignals zum Steuern der Beschränkungsvorrichtung.

**102.** Apparat nach Anspruch 101, wobei das Steuersignal ein frequenz-, amplituden- oder frequenz- oder amplitudenmoduliertes Signal umfasst.

**103.** Apparat nach Anspruch 102, wobei das Steuersignal ein analoges oder digitales Signal oder eine Kombination aus einem analogen und digitalen Signal umfasst.

**104.** Apparat nach einem der Ansprüche 101-103, wobei die Fernsteuerung in der Lage ist, Informationen über den Zustand der implantierbaren Beschränkungsvorrichtung zu erhalten und die Beschränkungsvorrichtung in Reaktion auf die Informationen zu steuern.

**105.** Apparat nach einem der Ansprüche 101-104, wobei die Fernsteuerung eine implantierbare Steuereinheit zum Steuern der Beschränkungsvorrichtung umfasst.

**106.** Apparat nach Anspruch 105, wobei die Steuereinheit einen Mikroprozessor umfasst.

**107.** Apparat nach einem der Ansprüche 101-106, wobei die drahtlose Fernsteuerung mindestens einen externen Signal-Sender oder -Sendeempfänger und mindestens einen internen Signal-Empfänger oder-Sendeempfänger umfasst, die im Patienten implantierbar sind.

**108.** Apparat nach einem der Ansprüche 100-106, wobei die drahtlose Fernsteuerung mindestens einen externen Signal-Empfänger oder-Sendeempfänger und mindestens einen internen Signal-Sender oder-Sendeempfänger umfasst, die im Patienten implantierbar sind.

**109.** Apparat nach einem der Ansprüche 101-108, wobei die Fernsteuerung in der Lage ist, Informationen bezogen auf die Beschränkungsvorrichtung vom Inneren des Patientenkörpers nach außerhalb davon zu senden.

**110.** Apparat nach Anspruch 109, wobei die Fernsteuerung die Beschränkungsvorrichtung in Reaktion auf die Informationen steuert.

**111.** Apparat nach einem der Ansprüche 101-110, wobei die Fernsteuerung einen Steuersignal-Sender zum Senden des drahtlosen Steuersignals umfasst, und die Energieübertragungsvorrichtung den Steuersignal-Sender umfasst, wobei Energie von dem Steuersignal übertragen wird.

**112.** Apparat nach einem der Ansprüche 101-110, wobei die Energieübertragungsvorrichtung Energie durch mindestens ein Signal separat von dem Steuersignal überträgt.

**113.** Apparat nach einem der Ansprüche 101-110, wobei die Fernsteuerung ein Trägersignal zum Tragen des Steuersignals übermittelt.

**114.** Apparat nach einem der Ansprüche 101-110, wobei die Energieübertragungsvorrichtung Energie durch mindestens ein Signal überträgt, das als Trägersignal für das Steuersignal verwendet wird, das von der Fernsteuerung übertragen wird.

**115.** Apparat nach Anspruch 114, wobei das Trägersignal frequenz-, amplituden- oder frequenz- und amplitudenmoduliert ist.

**116.** Apparat nach Anspruch 114 oder 115, wobei das Trägersignal digitale, analoge oder eine Kombination aus digitalen und analogen Signalen umfasst.

**117.** Apparat nach Anspruch 116, wobei die Signale Wellensignale umfassen.

**118.** Apparat nach einem der Ansprüche 101-117, wobei das Steuersignal ein Wellensignal umfasst, umfassend eines, ein Schall-Wellensignal, ein Ultraschall-Wellensignal, ein elektromagnetisches Wellensignal, ein Infrarot-Lichtsignal, ein sichtbares Lichtsignal, ein ultraviolettes Lichtsignal, ein Laser-Lichtsignal, ein Mikrowellensignal, ein Funkwellensignal, ein Röntgenstrahlen-Strahlungssignal oder ein Gammastrahlungssignal umfasst.

**119.** Apparat nach einem Ansprüche 101-117, wobei das Steuersignal ein elektrisches oder magnetisches Feld oder ein kombiniertes elektrisches und magnetisches Feld umfasst.

**120.** Apparat nach Anspruch 103, wobei die Fernsteuerung ein elektromagnetisches Trägerwellensignal zum Tragen des digitalen oder analogen Steuersignals überträgt.

**121.** Apparat nach Anspruch 1, wobei die Energie der zweiten Form, die zum Betreiben der Beschränkungsvorrichtung verwendet wird, drahtlos von der Energieumwandlungsvorrichtung übertragen wird.

**122.** Apparat nach Anspruch 1, überdies umfassend eine implantierbare Steuereinheit zum Steuern der Beschränkungsvorrichtung.

**123.** Apparat nach Anspruch 120, wobei die Steuereinheit programmierbar zum Steuern der Beschränkungsvorrichtung gemäß einem Programm ist.

**124.** Apparat nach Anspruch 123, wobei die Steuereinheit die Beschränkungsvorrichtung über die Zeit gemäß einem Aktivitätsplanprogramm steuert.

**125.** Apparat nach einem der Ansprüche 122- 124, überdies umfassend eine externe drahtlose Fernsteuerung zum Programmieren der implantierbaren Steuereinheit.

**126.** Apparat nach Anspruch 1, überdies umfassend einen externen Datenkommunikator und einen implantierbaren internen Datenkommunikator, der mit dem externen Datenkommunikator kommuniziert, wobei der interne Kommunikator Daten, die auf die Beschränkungsvorrichtung bezogen sind, zurück zum externen Datenkommunikator führt oder der externe Datenkommunikator Daten zum internen Datenkommunikator führt.

**127.** Apparat nach Anspruch 126, wobei der interne Datenkommunikator Daten zuführt, die auf mindestens ein physikalisches Signal des Patienten bezogen sind.

**128.** Apparat nach einem der vorhergehenden Ansprüche, wobei die Beschränkungsvorrichtung angepasst ist, wenn implantiert, die Beschränkung des Fäkal-Durchgangs zu steuern.

**129.** Apparat nach einem der vorhergehenden Ansprüche, wobei die Beschränkungsvorrichtung nicht aufblasbar ist.

**130.** Apparat nach Anspruch 1, wobei eine, die Energie der ersten Form oder die Energie der zweiten Form, magnetische Energie, kinetische Energie, Schallenergie, chemische Energie, Strahlungsenergie, elektromagnetische Energie, Lichtenergie, Nuklearenergie oder thermische Energie umfasst.

**131.** Apparat nach Anspruch 1, wobei eine, die Energie der ersten Form oder die Energie der zweiten Form, nicht magnetisch, nicht kinetisch, nicht chemisch, nicht schallenergetisch, nicht nuklear oder nicht thermisch ist.

**132.** Apparat nach Anspruch 1, wobei die Energieübertragungsvorrichtung anders als die Energieumwandlungsvorrichtung funktioniert.

**133.** Apparat nach Anspruch 1, wobei die Energieübertragungsvorrichtung ähnlich wie die Energieumwandlungsvorrichtung funktioniert.

**134.** Apparat nach einem der vorhergehenden Ansprüche, wobei die Energieumwandlungsvorrichtung ausgelegt ist, subkutan oder im Abdomen, Thorax- oder Zephalbereich des Patienten implantiert zu werden.

**135.** Apparat nach einem der Ansprüche 1-134, wobei die Energieumwandlungsvorrichtung ausgelegt ist, in einer Öffnung des Patientenkörpers und unter der Schleimhaut oder intraluminar außerhalb der Schleimhaut der Öffnung implantiert zu werden.

**136.** Apparat nach einem der vorhergehenden Ansprüche, wobei die Beschränkungsvorrichtung in einem weichen oder gelartigen Material eingebettet ist.

**137.** Apparat nach einem der vorhergehenden Ansprüche, wobei die Beschränkungsvorrichtung in einem Silikonmaterial eingebettet ist, das eine Härte von weniger als 20 Shore aufweist.

## Revendications

**1.** Appareil de traitement de la maladie d'incontinence anale pour le traitement d'un patient qui souffre d'incontinence anale, comprenant
un dispositif de restriction (4) implantable dans le patient pour occuper le colon, le rectum ou l'anus pour former un passage fécal restreint dans celui-ci, le dispositif de restriction pouvant fonctionner pour effectuer une fonction réversible impliquant l'élargissement et la restriction du passage fécal,
un dispositif de fonctionnement (15, 18, 32, 128 ; 18, 32) implantable dans le patient pour faire fonctionner le dispositif de restriction,

un dispositif de transmission d'énergie (10) pour une transmission d'énergie sans fil de l'extérieur du corps du patient à l'intérieur du corps du patient pour l'utilisation en connexion avec le fonctionnement du dispositif de restriction comprenant l'actionnement du dispositif de fonctionnement, et
un dispositif de transformation d'énergie (6) implantable dans le patient pour transformer l'énergie de la première forme transmise sans fil par le dispositif de transmission d'énergie en une énergie d'une seconde forme différente de l'énergie de la première forme, le dispositif de restriction pouvant fonctionner en réaction à l'énergie de la seconde forme pour changer la restriction du passage fécal, quand le dispositif de restriction est implanté, dans lequel le dispositif de fonctionnement comprend :

(i) un moteur (15, 18, 32, 128) implantable dans le patient, l'énergie de la seconde forme étant utilisée pour actionner le moteur pour faire fonctionner de manière réversible le dispositif de restriction (4) ; ou

une pompe (18, 32) implantable dans le patient, l'énergie de la seconde forme étant utilisée pour actionner la pompe pour faire fonctionner le dispositif de restriction (4), **caractérisé en ce que** l'appareil comprend en outre au moins une surveillance de niveau de tension implantable.

**2.** Appareil selon la revendication 1, dans lequel le dispositif de transformation d'énergie comprend au moins un élément présentant une région positive et une région négative, l'élément est capable de créer un champ énergétique entre les régions positive et négative quand il est soumis à l'énergie de la première forme transmise par le dispositif de transmission d'énergie, et le champ énergétique produit l'énergie de la seconde forme.

**3.** Appareil selon la revendication 2, dans lequel l'élément comprend un élément de jonction électrique, et l'élément de jonction électrique est capable d'induire un champ électrique entre les régions positive et négative quand il est soumis à l'énergie de la première forme transmise par le dispositif de transmission d'énergie, ce par quoi l'énergie de la seconde forme comprend de l'énergie électrique.

**4.** Appareil selon la revendication 3, dans lequel le dispositif de restriction est à fonctionnement électrique, et les régions positive et négative de l'élément de jonction électrique alimentent de l'énergie électrique pour le fonctionnement du dispositif de restriction.

**5.** Appareil selon la revendication 4, comprenant en outre des conducteurs électriques connectés aux régions positive et négative de l'élément de jonction électrique, ce par quoi l'élément de jonction électrique est capable d'alimenter un courant électrique par les conducteurs.

**6.** Appareil selon la revendication 5, dans lequel l'élément de jonction électrique est capable d'alimenter un courant continu ou un courant continu pulsé par les conducteurs.

**7.** Appareil selon la revendication 5, dans lequel l'élément de jonction électrique est capable d'alimenter un courant alternatif ou une combinaison d'un courant continu et alternatif par les conducteurs.

**8.** Appareil selon la revendication 4, dans lequel l'élément de jonction électrique est capable d'alimenter un signal modulé en fréquence ou en amplitude.

**9.** Appareil selon la revendication 4, dans lequel l'élément de jonction électrique est capable d'alimenter un signal analogique ou numérique.

**10.** Appareil selon l'une quelconque des revendications 1 - 9, dans lequel le dispositif de transformation d'énergie forme une feuille plate et fine, et présente un volume de moins de 2000 $cm^3$.

**11.** Appareil selon la revendication 1, dans lequel le dispositif de transformation d'énergie est adapté pour transformer l'énergie de la première forme directement ou indirectement en énergie de la seconde forme.

**12.** Appareil selon la revendication 1, dans lequel le dispositif de transformation d'énergie est adapté pour actionner directement le moteur ou la pompe par l'énergie transformée, lorsque l'énergie de la seconde forme est en train d'être transformée à partir de l'énergie de la première forme.

**13.** Appareil selon l'une quelconque des revendications 11 - 12, dans lequel l'énergie sans fil de la première forme comprend des ondes sonores et l'énergie de la seconde forme comprend de l'énergie électrique.

**14.** Appareil selon l'une quelconque des revendications 1 - 13, dans lequel le dispositif de transformation d'énergie comprend un condensateur et l'énergie de la seconde forme comprend de l'énergie électrique.

**15.** Appareil selon la revendication 14, dans lequel le condensateur est adapté pour produire des impulsions électriques à partir de l'énergie électrique transformée.

**16.** Appareil selon la revendication 15, dans lequel le condensateur est adapté pour produire les impulsions de l'énergie électrique, lorsque le dispositif de transformation d'énergie transforme l'énergie de la première forme transmise par le dispositif de transmission d'énergie en énergie électrique de la seconde forme.

**17.** Appareil selon la revendication 1, comprenant en outre un stabilisateur implantable pour stabiliser l'énergie de la seconde forme.

**18.** Appareil selon la revendication 17, dans lequel l'énergie de la seconde forme comprend un courant électrique et le stabilisateur comprend au moins un condensateur.

**19.** Appareil selon la revendication 1, comprenant en outre des composants électriques implantables incluant un condensateur ou un accumulateur.

**20.** Appareil selon l'une quelconque des revendications 1 - 19, comprenant en outre des composants électriques implantables incluant une unique surveillance de niveau de tension.

**21.** Appareil selon la revendication 19 ou 20, dans lequel les composants électriques sont dépourvus de tout détecteur de courant et/ou détecteur de niveau de charge.

**22.** Appareil selon l'une quelconque des revendications 19 - 21, comprenant en outre un condensateur ou accumulateur implantable, dans lequel la charge ou la décharge du condensateur ou de l'accumulateur est commandée par l'utilisation de la surveillance de niveau de tension.

**23.** Appareil selon l'une quelconque des revendications 14 - 16, 18 et 22, dans lequel le condensateur présente une capacité de moins de 0,1 $\mu$F.

**24.** Appareil selon la revendication 1, dans lequel le dispositif de transmission d'énergie est adapté pour transmettre de l'énergie sans fil pour l'utilisation directe en connexion avec le fonctionnement du dispositif de restriction, lorsque l'énergie sans fil est en train d'être transmise.

**25.** Appareil selon la revendication 24, dans lequel le dispositif de transmission d'énergie est adapté pour actionner directement le moteur ou la pompe avec de l'énergie sans fil.

**26.** Appareil selon la revendication 25, dans lequel le dispositif de transmission d'énergie est adapté pour transmettre de l'énergie sans fil sous forme d'un champ magnétique ou d'ondes électromagnétiques pour l'actionnement direct du moteur ou de la pompe.

**27.** Appareil selon la revendication 1, dans lequel le dispositif de transformation d'énergie est adapté pour alimenter l'énergie de la seconde forme pour l'utilisation directe en connexion avec le fonctionnement du dispositif de restriction, lorsque l'énergie de la première forme est en train d'être transformée en énergie de la seconde forme.

**28.** Appareil selon la revendication 27, dans lequel le dispositif de transformation d'énergie est adapté pour actionner directement le moteur ou la pompe avec l'énergie de la seconde forme.

**29.** Appareil selon la revendication 28, dans lequel le dispositif de transformation d'énergie fait fonctionner directement le dispositif de restriction avec l'énergie de la seconde forme de manière non magnétique, non thermique ou non mécanique.

**30.** Appareil selon l'une quelconque des revendications 1, dans lequel le moteur fait fonctionner directement ou par intermittence le dispositif de restriction, et le dispositif de transformation d'énergie actionne le moteur avec l'énergie de la seconde forme.

**31.** Appareil selon l'une quelconque des revendications 1 - 24 et 27, dans lequel le dispositif de restriction comprend un dispositif de restriction hydraulique, et comprenant en outre une pompe implantable pour faire fonctionner le dispositif de restriction hydraulique, le dispositif de transformation d'énergie alimentant l'énergie de la seconde forme pour entraîner la pompe.

**32.** Appareil selon l'une quelconque des revendications 25, 26, 28 et 31, dans lequel la pompe n'est pas une pompe de type à piston plongeur.

**33.** Appareil selon l'une quelconque des revendications précédentes, dans lequel le dispositif de transformation d'énergie est capable de générer en tant qu'énergie de la seconde forme un courant dépassant 1 μA, quand il transfère l'énergie de la première forme transmise par le dispositif de transmission d'énergie.

**34.** Appareil selon l'une quelconque des revendications précédentes, comprenant en outre un dispositif d'ajustage pour ajuster le dispositif de restriction pour changer la restriction du passage fécal, dans lequel le dispositif d'ajustage est adapté pour ajuster mécaniquement le dispositif de restriction, ou adapté pour ajuster hydrauliquement le dispositif de restriction en utilisant un moyen hydraulique qui est dépourvu de fluide hydraulique du type présentant une viscosité qui augmente sensiblement quand il est soumis à la chaleur ou à un champ magnétique.

**35.** Appareil selon l'une quelconque des revendications 1 - 10, dans lequel le dispositif de transformation d'énergie comprend au moins un composant de type semi-conducteur.

**36.** Appareil selon la revendication 35, dans lequel le dispositif de transformation d'énergie comprend des circuits de composants semi-conducteurs.

**37.** Appareil selon la revendication 35, dans lequel le composant semi-conducteur comprend un transistor ou une puce ou des composants électroniques similaires à l'exception de diodes de redressement.

**38.** Appareil selon la revendication 36 ou 37, dans lequel le composant semi-conducteur comprend au moins un élément présentant une région positive et une région négative, l'élément est capable de créer un champ énergétique entre les régions positive et négative quand il est soumis à l'énergie de la première forme transmise par le dispositif de transmission d'énergie, et le champ énergétique produit l'énergie de la seconde forme.

**39.** Appareil selon la revendication 1, comprenant en outre un dispositif de commande pour commander le moteur.

**40.** Appareil selon la revendication 29 ou 39, comprenant en outre un engrenage implantable connecté au moteur.

**41.** Appareil selon les revendications 39 et 40, dans lequel le moteur comprend un moteur rotatif et le dispositif de commande commande le moteur rotatif pour le faire tourner un nombre de tours souhaité.

**42.** Appareil selon la revendication 39, dans lequel le moteur comprend un moteur linéaire.

**43.** Appareil selon la revendication 39 et 40, dans lequel le moteur comprend un moteur à fluide hydraulique ou pneumatique, et le dispositif de commande commande le moteur à fluide.

**44.** Appareil selon la revendication 39, dans lequel le moteur comprend un moteur électrique présentant des pièces électriquement conductrices fabriquées en matière plastique.

**45.** Appareil selon la revendication 39, dans lequel le dispositif de restriction comprend un moyen hydraulique.

**46.** Appareil selon la revendication 45, comprenant en outre un conduit à fluide connecté au moyen hydraulique du dispositif de restriction, et un réservoir à fluide, le réservoir formant une partie du conduit.

**47.** Appareil selon la revendication 46, dans lequel le moyen hydraulique et le conduit sont dépourvus de tout clapet anti-retour.

**48.** Appareil selon la revendication 47, dans lequel le réservoir forme une chambre à fluide avec un volume variable, et le moteur est adapté pour distribuer du fluide de la chambre au moyen hydraulique du dispositif de restriction en réduisant le volume de la chambre et pour retirer du fluide du moyen hydraulique à la chambre en augmentant le volume de la chambre.

**49.** Appareil selon la revendication 48, dans lequel le dispositif de fonctionnement comprend un moteur implantable utilisé pour réduire et augmenter le volume de la chambre.

**50.** Appareil selon l'une quelconque des revendications 45 - 48, dans lequel la pompe est adaptée pour pomper le fluide hydraulique dans le moyen hydraulique du dispositif de restriction.

**51.** Appareil selon la revendication 39, dans lequel le dispositif de commande inverse la polarité de l'énergie de la seconde forme pour inverser la marche du moteur.

**52.** Appareil selon la revendication 39 ou 49, dans lequel le moteur comprend un moteur électrique et l'énergie de la seconde forme comprend de l'énergie électrique.

**53.** Appareil selon la revendication 52, comprenant en outre un dispositif de renversement implantable dans le patient pour renverser la fonction effectuée par le dispositif de restriction.

**54.** Appareil selon les revendications 39 et 53, dans lequel le dispositif de commande commande le dispositif de renversement pour renverser la fonction effectuée par le dispositif de restriction.

**55.** Appareil selon la revendication 53 ou 54, dans lequel le dispositif de renversement comprend un moyen hydraulique incluant une soupape pour inverser la direction d'écoulement d'un écoulement de fluide dans le moyen hydraulique.

**56.** Appareil selon la revendication 53 ou 54, dans lequel le dispositif de renversement comprend un dispositif de renversement mécanique.

**57.** Appareil selon la revendication 56, dans lequel le dispositif de renversement comprend une boîte de transmission.

**58.** Appareil selon la revendication 53 ou 54, dans lequel le dispositif de renversement comprend un interrupteur.

**59.** Appareil selon la revendication 58, dans lequel l'interrupteur peut fonctionner par l'énergie de la seconde forme.

**60.** Appareil selon la revendication 59, dans lequel le dispositif de commande commande le fonctionnement de l'interrupteur en inversant la polarité de l'énergie de la seconde forme.

**61.** Appareil selon la revendication 58 ou 59, dans lequel l'interrupteur comprend un interrupteur électrique et l'énergie de la seconde forme comprend de l'énergie électrique.

**62.** Appareil selon l'une quelconque des revendications 15, 16 et 39, dans lequel le dispositif de commande est adapté pour commander le dispositif de transformation d'énergie pour produire l'énergie de la seconde forme dans une succession d'impulsions énergétiques pour l'utilisation directe en connexion avec le fonctionnement du dispositif de restriction.

**63.** Appareil selon la revendication 1, comprenant en outre un dispositif de stockage d'énergie implantable dans le patient pour stocker l'énergie de la seconde forme et pour alimenter de l'énergie en connexion avec le fonctionnement du dispositif de restriction.

**64.** Appareil selon la revendication 63, dans lequel le dispositif de stockage d'énergie comprend un accumulateur.

**65.** Appareil selon la revendication 64, dans lequel l'énergie de la seconde forme comprend de l'énergie électrique et le dispositif de stockage d'énergie comprend un accumulateur électrique.

**66.** Appareil selon la revendication 65, dans lequel l'accumulateur électrique comprend au moins un condensateur ou au moins une pile rechargeable, ou une combinaison d'au moins un condensateur et d'au moins une pile rechargeable.

**67.** Appareil selon l'une quelconque des revendications 1, 58, 63 - 66, comprenant en outre un interrupteur implantable dans le patient pour commuter directement ou indirectement le fonctionnement du dispositif de restriction.

**68.** Appareil selon la revendication 67, comprenant en outre une source d'énergie implantable dans le patient, dans lequel l'énergie de la seconde forme alimentée par le dispositif de stockage d'énergie fait fonctionner l'interrupteur pour commuter d'un mode d'arrêt, dans lequel la source d'énergie n'est pas utilisée, à un mode de marche, dans lequel la source d'énergie alimente de l'énergie pour le fonctionnement du dispositif de restriction.

**69.** Appareil selon la revendication 67, comprenant en outre une source d'énergie implantable dans le patient, et une commande à distance pour commander l'alimentation en énergie de la source d'énergie, dans lequel l'énergie de la seconde forme alimentée par le dispositif de stockage d'énergie fait fonctionner l'interrupteur pour commuter d'un mode d'arrêt, dans lequel la commande à distance ne peut pas commander la source d'énergie et la source d'énergie n'est pas utilisée, à un mode de veille, dans lequel la commande à distance est autorisée à commander la source d'énergie pour alimenter de l'énergie pour le fonctionnement du dispositif de restriction.

**70.** Appareil selon la revendication 67, comprenant en outre une source d'énergie implantable dans le patient pour alimenter de l'énergie pour le fonctionnement du dispositif de restriction, dans lequel l'énergie de la seconde forme alimentée par le dispositif de transformation d'énergie fait fonctionner l'interrupteur pour commuter d'un mode d'arrêt, dans lequel la source d'énergie n'est pas utilisée, à un mode de marche, dans lequel la source d'énergie alimente de l'énergie pour le fonctionnement du dispositif de restriction.

**71.** Appareil selon la revendication 67, comprenant en outre une source d'énergie implantable dans le patient pour alimenter de l'énergie pour le fonctionnement du dispositif de restriction, et une commande à distance pour commander l'alimentation en énergie de la source d'énergie implantable, dans lequel l'énergie de la seconde forme alimentée par le dispositif de transformation d'énergie fait fonctionner l'interrupteur pour commuter d'un mode d'arrêt, dans lequel la commande à distance ne peut pas commander la source d'énergie et la source d'énergie n'est pas utilisée, à un mode de veille, dans lequel la commande à distance est autorisée à commander la source d'énergie pour alimenter de l'énergie pour le fonctionnement du dispositif de restriction.

**72.** Appareil selon la revendication 67, comprenant en outre une source d'énergie implantable dans le patient pour alimenter de l'énergie pour le fonctionnement du dispositif de restriction, dans lequel l'énergie de la première forme alimentée par le dispositif de transmission d'énergie fait fonctionner l'interrupteur pour commuter d'un mode d'arrêt, dans lequel la source d'énergie n'est pas utilisée, à un mode de marche, dans lequel la source d'énergie alimente de l'énergie pour le fonctionnement du dispositif de restriction.

**73.** Appareil selon la revendication 67, comprenant en outre une source d'énergie implantable dans le patient pour alimenter de l'énergie pour le fonctionnement du dispositif de restriction, et une commande à distance pour commander l'alimentation en énergie de la source d'énergie implantable, dans lequel l'énergie de la première forme alimentée par le dispositif de transmission d'énergie fait fonctionner l'interrupteur pour commuter d'un mode d'arrêt, dans lequel la commande à distance ne peut pas commander la source d'énergie et la source d'énergie n'est pas utilisée, à un mode de veille, dans lequel la commande à distance est autorisée à commander la source d'énergie pour alimenter de l'énergie pour le fonctionnement du dispositif de restriction.

**74.** Appareil selon la revendication 1, dans lequel le dispositif de restriction est à fonctionnement électrique, et l'énergie de la seconde forme comprend de l'énergie électrique.

**75.** Appareil selon la revendication 74, comprenant en outre des conducteurs électriques connectés au dispositif de transformation d'énergie, ce par quoi le dispositif de transformation d'énergie est capable d'alimenter un courant électrique par les conducteurs.

**76.** Appareil selon la revendication 1, dans lequel le dispositif de transformation d'énergie est capable d'alimenter un signal modulé en fréquence, en amplitude ou en fréquence et en amplitude.

**77.** Appareil selon la revendication 1, dans lequel le dispositif de transformation d'énergie est capable d'alimenter un signal analogique, numérique ou une combinaison de signal analogique et numérique.

**78.** Appareil selon la revendication 1, comprenant en outre une source d'énergie activable implantable dans le patient, dans lequel la source d'énergie est activée par de l'énergie sans fil transmise par le dispositif de transmission d'énergie, pour alimenter de l'énergie qui est utilisée en connexion avec le fonctionnement du dispositif de restriction.

**79.** Appareil selon la revendication 1, dans lequel le dispositif de transmission d'énergie transmet de l'énergie par au moins un signal sans fil.

**80.** Appareil selon la revendication 79, dans lequel le signal contient de l'énergie rayonnante.

**81.** Appareil selon la revendication 80, dans lequel le signal comprend un signal d'ondes.

**82.** Appareil selon la revendication 81, dans lequel le signal d'ondes comprend un signal d'ondes électromagnétiques incluant l'un parmi un signal lumineux infrarouge, un signal lumineux visible, un signal lumineux ultraviolet, un signal laser, un signal à micro-ondes, un signal d'ondes radio, un signal de rayonnement de rayons X, et un signal de rayonnement gamma.

**83.** Appareil selon la revendication 82, dans lequel le signal d'ondes comprend un signal d'ondes sonores ou ultrasonores.

**84.** Appareil selon l'une quelconque des revendications 79 - 83, dans lequel le signal comprend un signal numérique ou analogique, ou une combinaison d'un signal numérique et analogique.

**85.** Appareil selon la revendication 1, dans lequel l'énergie de la première forme transmise par le dispositif de transmission d'énergie comprend un champ électrique, électromagnétique ou magnétique, ou une combinaison de ceux-ci.

**86.** Appareil selon la revendication 85, dans lequel le champ électrique, électromagnétique ou magnétique, ou la combinaison de ceux-ci est transmis sous forme d'impulsions ou d'impulsions numériques, ou une combinaison d'impulsions et d'impulsions numériques par le dispositif de transmission d'énergie.

**87.** Appareil selon la revendication 1, dans lequel l'énergie d'une première forme transmise par le dispositif de transmission d'énergie comprend un champ électrique, électromagnétique ou magnétique, ou une combinaison de ceux-ci.

**88.** Appareil selon la revendication 87, dans lequel le champ électrique, électromagnétique ou magnétique, ou la combinaison de ceux-ci est transmis sous forme d'ondes ou d'impulsions analogiques ou une combinaison de celles-ci par le dispositif de transmission d'énergie.

**89.** Appareil selon l'une quelconque des revendications 1 - 88, dans lequel l'énergie transmise par le dispositif de transmission d'énergie comprend de l'énergie polarisée.

**90.** Appareil selon la revendication 1, dans lequel le dispositif de transformation d'énergie transforme l'énergie de la première forme en un courant continu ou un courant continu pulsé, ou une combinaison d'un courant continu et d'un courant continu pulsé.

**91.** Appareil selon la revendication 1, dans lequel le dispositif de transformation d'énergie transforme l'énergie de la première forme en un courant alternatif ou une combinaison d'un courant continu et alternatif.

**92.** Appareil selon la revendication 1, comprenant en outre un générateur d'impulsions implantable pour générer des impulsions électriques à partir de l'énergie de la seconde forme produite par le champ énergétique.

**93.** Appareil selon l'une quelconque des revendications précédentes, comprenant en outre au moins un capteur implantable pour capter au moins un paramètre physique du patient.

**94.** Appareil selon la revendication 93, dans lequel le capteur comprend un capteur de pression pour capter directement ou indirectement en tant que paramètre physique la pression dans le passage fécal où la pression contre le colon, le rectum ou l'anus.

**95.** Appareil selon la revendication 94, comprenant en outre un dispositif de commande pour commander le dispositif de restriction en réaction à des signaux du capteur.

**96.** Appareil selon la revendication 95, dans lequel le dispositif de commande comprend une unité de commande interne implantable dans le patient pour commander le dispositif de restriction en réaction à des signaux du capteur.

**97.** Appareil selon la revendication 96, dans lequel l'unité de commande interne commande directement le dispositif de restriction en réaction à des signaux du capteur.

**98.** Appareil selon la revendication 97, dans lequel le dispositif de commande comprend une unité de commande externe à l'extérieur du corps du patient pour commander le dispositif de restriction en réaction à des signaux du capteur.

**99.** Appareil selon la revendication 98, dans lequel l'unité de commande externe stocke des informations sur le paramètre physique capté par le capteur et est à fonctionnement manuel pour commander le dispositif de restriction sur la base des informations stockées.

**100.** Appareil selon l'une quelconque des revendications 93 - 99, comprenant en outre au moins un émetteur implantable pour émettre des informations sur le paramètre physique capté par le capteur.

**101.** Appareil selon l'une quelconque des revendications précédentes, comprenant en outre une commande à distance sans fil pour transmettre au moins un signal de commande sans fil pour commander le dispositif de restriction.

**102.** Appareil selon la revendication 101, dans lequel le signal de commande comprend un signal modulé en fréquence, en amplitude ou en fréquence ou amplitude.

**103.** Appareil selon la revendication 102, dans lequel le signal de commande comprend un signal analogique ou numérique, ou une combinaison d'un signal analogique et numérique.

**104.** Appareil selon l'une quelconque des revendications 101 - 103, dans lequel la commande à distance est capable d'obtenir des informations sur la condition du dispositif de restriction implantable et de commander le dispositif de restriction en réaction aux informations.

**105.** Appareil selon l'une quelconque des revendications 101 - 104, dans lequel la commande à distance comprend une unité de commande implantable pour commander le dispositif de restriction.

**106.** Appareil selon la revendication 105, dans lequel l'unité de commande comprend un microprocesseur.

**107.** Appareil selon l'une quelconque des revendications 101 - 106, dans lequel la commande à distance sans fil comprend au moins un émetteur ou émetteur-récepteur de signal externe et au moins un récepteur ou émetteur-récepteur de signal interne implantables dans le patient.

**108.** Appareil selon l'une quelconque des revendications 100 - 106, dans lequel la commande à distance sans fil comprend au moins un récepteur ou émetteur-récepteur de signal externe et au moins un émetteur ou émetteur-récepteur de signal interne implantables dans le patient.

**109.** Appareil selon l'une quelconque des revendications 101 - 108, dans lequel la commande à distance est capable d'envoyer des informations relatives au dispositif de restriction de l'intérieur du corps du patient à l'extérieur de celui-ci.

**110.** Appareil selon la revendication 109, dans lequel la commande à distance commande le dispositif de restriction en réaction aux informations.

**111.** Appareil selon l'une quelconque des revendications 101 - 110, dans lequel la commande à distance comprend un émetteur de signal de commande pour transmettre le signal de commande sans fil, et le dispositif de transmission d'énergie comprend l'émetteur de signal de commande, ce par quoi de l'énergie est transmise par le signal de commande.

**112.** Appareil selon l'une quelconque des revendications 101 - 110, dans lequel le dispositif de transmission d'énergie transmet de l'énergie par au moins un signal séparé du signal de commande.

**113.** Appareil selon l'une quelconque des revendications 101 - 110, dans lequel la commande à distance transmet un signal porteur pour porter le signal de commande.

**114.** Appareil selon l'une quelconque des revendications 101 - 110, dans lequel le dispositif de transmission d'énergie transmet de l’énergie par au moins un signal, qui est utilisé en tant que signal porteur pour le signal de commande transmis par la commande à distance.

**115.** Appareil selon la revendication 114, dans lequel le signal porteur est modulé en fréquence, en amplitude ou en fréquence et amplitude.

**116.** Appareil selon la revendication 114 ou 115, dans lequel le signal porteur comprend un signal numérique, analogique ou une combinaison de signaux numérique et analogique.

**117.** Appareil selon la revendication 116, dans lequel les signaux comprennent des signaux d’ondes.

**118.** Appareil selon l’une quelconque des revendications 101 - 117, dans lequel le signal de commande comprend un signal d’ondes comprenant l’un parmi un signal d’ondes sonores, un signal d’ondes ultrasonores, un signal d’ondes électromagnétiques, un signal lumineux infrarouge, un signal lumineux visible, un signal lumineux ultraviolet, un signal lumineux laser, un signal à micro-ondes, un signal d’ondes radio, un signal de rayonnement de rayons X et un signal de rayonnement gamma.

**119.** Appareil selon l’une quelconque des revendications 101 - 117, dans lequel le signal de commande comprend un champ électrique ou magnétique, ou un champ combiné électrique et magnétique.

**120.** Appareil selon la revendication 103, dans lequel la commande à distance transmet un signal d’ondes porteuses électromagnétiques pour porter le signal de commande numérique ou analogique.

**121.** Appareil selon la revendication 1, dans lequel l’énergie de la seconde forme utilisée pour faire fonctionner le dispositif de restriction est transmise sans fil par le dispositif de transformation d’énergie.

**122.** Appareil selon la revendication 1, comprenant en outre une unité de commande implantable pour commander le dispositif de restriction.

**123.** Appareil selon la revendication 120, dans lequel l’unité de commande est programmable pour commander le dispositif de restriction conformément à un programme.

**124.** Appareil selon la revendication 123, dans lequel l’unité de commande commande le dispositif de restriction sur la durée conformément à un programme de déroulement des activités.

**125.** Appareil selon l’une quelconque des revendications 122 - 124, comprenant en outre une commande à distance sans fil externe pour programmer l’unité de commande implantable.

**126.** Appareil selon la revendication 1, comprenant en outre un communicateur de données externe et un communicateur de données interne implantable communiquant avec le communicateur de données externe, dans lequel le communicateur interne renvoie des données relatives au dispositif de restriction au communicateur de données externe ou le communicateur de données externe fournit des données au communicateur de données interne.

**127.** Appareil selon la revendication 126, dans lequel le communicateur de données interne fournit des données relatives à au moins un signal physique du patient.

**128.** Appareil selon l’une quelconque des revendications précédentes, dans lequel le dispositif de restriction est adapté pour commander la restriction du passage fécal quand il est implanté.

**129.** Appareil selon l’une quelconque des revendications précédentes, dans lequel le dispositif de restriction est non gonflable.

**130.** Appareil selon la revendication 1, dans lequel l’une parmi l’énergie de la première forme et l’énergie de la seconde forme comprend de l’énergie magnétique, de l’énergie cinétique, de l’énergie sonore, de l’énergie chimique, de l’énergie rayonnante, de l’énergie électromagnétique, de la photoénergie, de l’énergie nucléaire ou de l’énergie thermique.

**131.** Appareil selon la revendication 1, dans lequel l’une parmi l’énergie de la première forme et l’énergie de la seconde forme est non magnétique, non cinétique, non chimique, non sonore, non nucléaire ou non thermique.

**132.** Appareil selon la revendication 1, dans lequel le dispositif de transmission d'énergie fonctionne différemment du dispositif de transformation d'énergie.

**133.** Appareil selon la revendication 1, dans lequel le dispositif de transmission d'énergie fonctionne de manière similaire au dispositif de transformation d'énergie.

**134.** Appareil selon l'une quelconque des revendications précédentes, dans lequel le dispositif de transformation d'énergie est conçu pour être implanté de manière sous-cutanée ou dans la région de l'abdomen, thoracique, ou céphalique du patient.

**135.** Appareil selon l'une quelconque des revendications 1 - 134, dans lequel le dispositif de transformation d'énergie est conçu pour être implanté dans un orifice du corps du patient et sous la muqueuse ou de manière endoluminale à l'extérieur de la muqueuse de l'orifice.

**136.** Appareil selon l'une quelconque des revendications précédentes, dans lequel le dispositif de restriction est enfoncé dans un matériau mou ou ayant la consistance d'un gel.

**137.** Appareil selon l'une quelconque des revendications précédentes, dans lequel le dispositif de restriction est enfoncé dans un matériau en silicone présentant une dureté inférieure à 20 Shore.

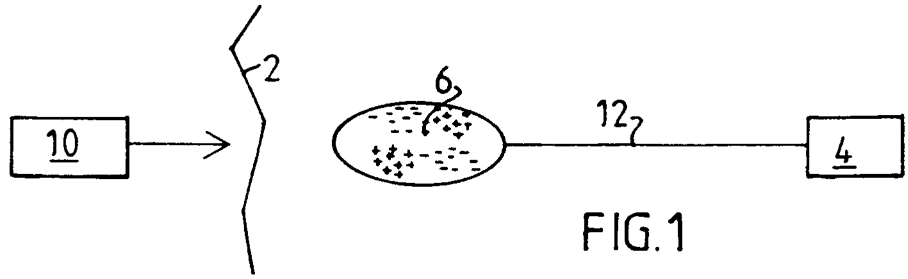


FIG.1

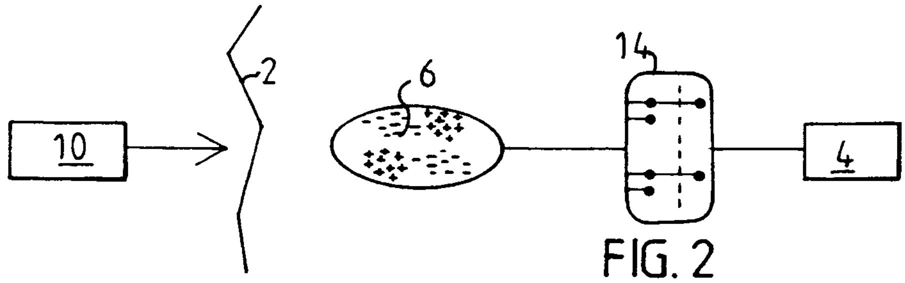


FIG. 2

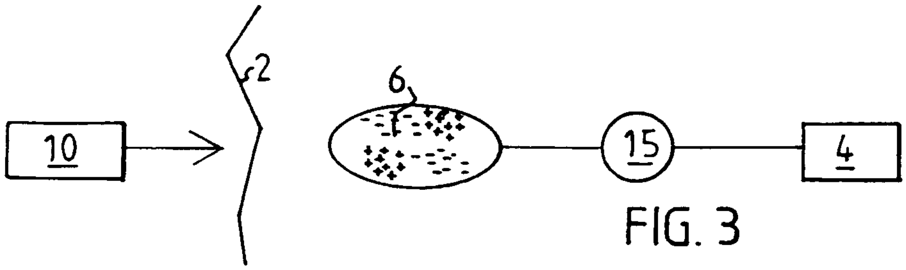


FIG. 3

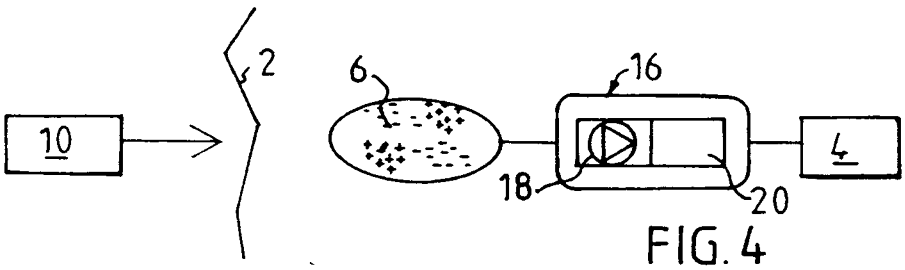


FIG. 4

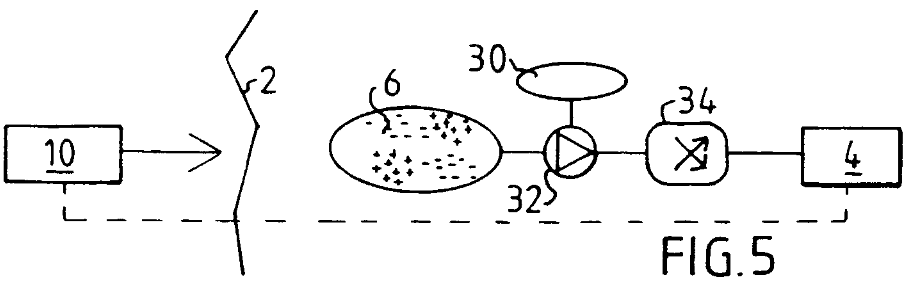


FIG.5

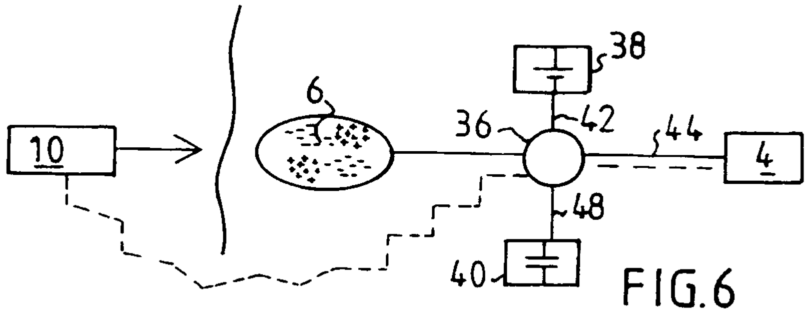

FIG.6

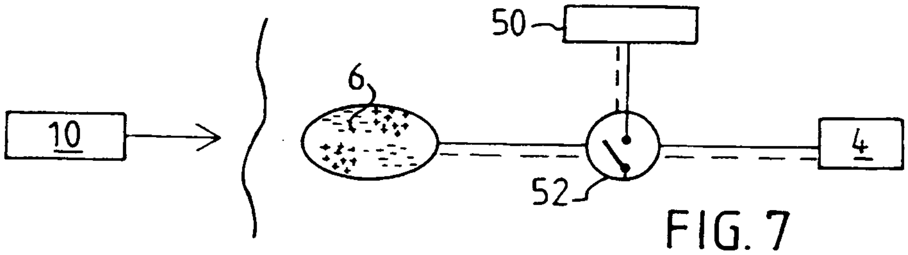

FIG. 7

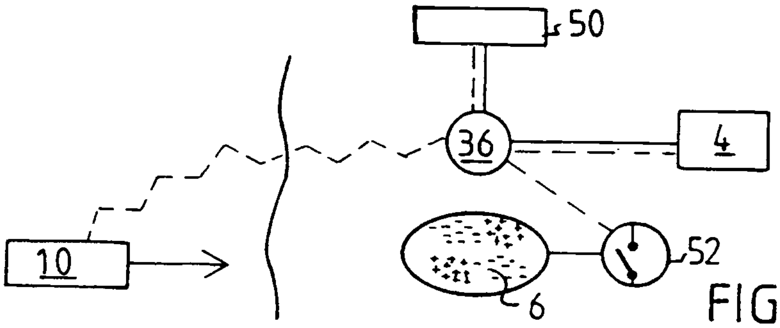

FIG.8

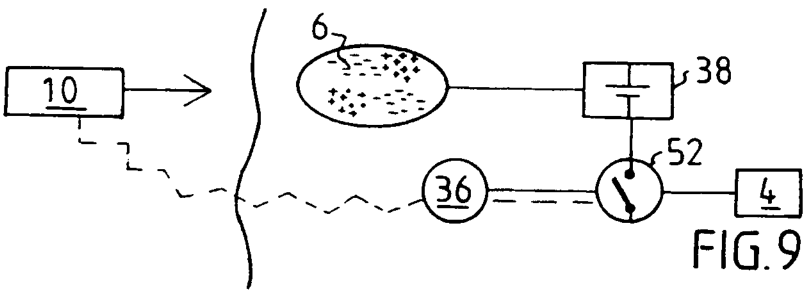

FIG.9

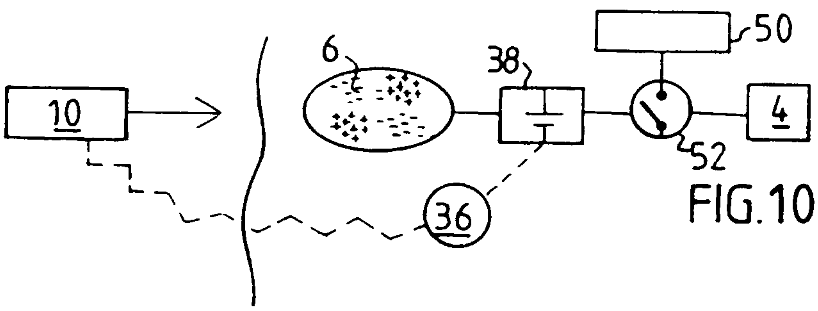

FIG.10

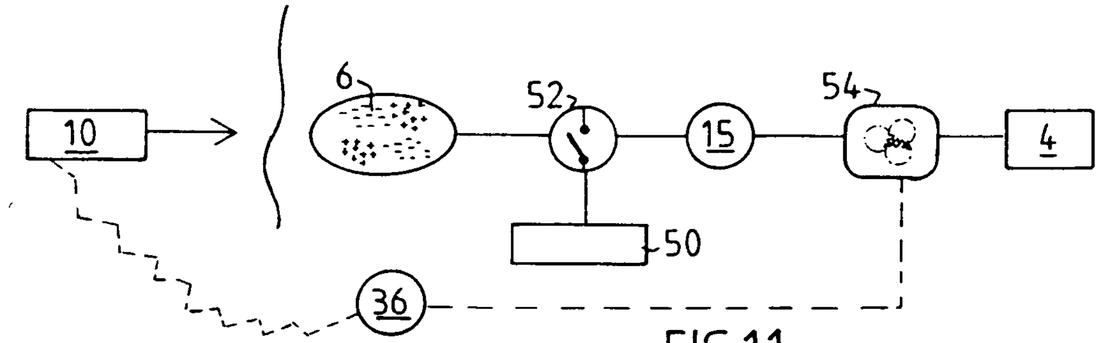


FIG.11

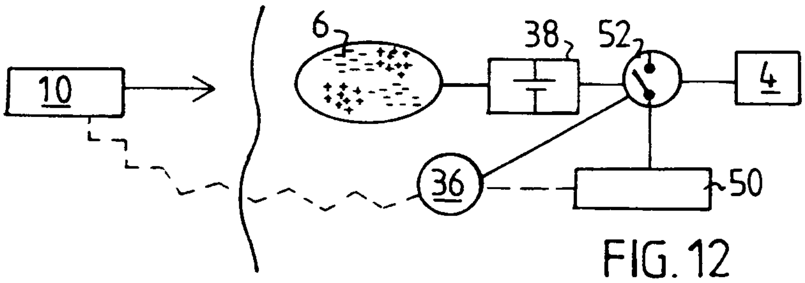


FIG. 12

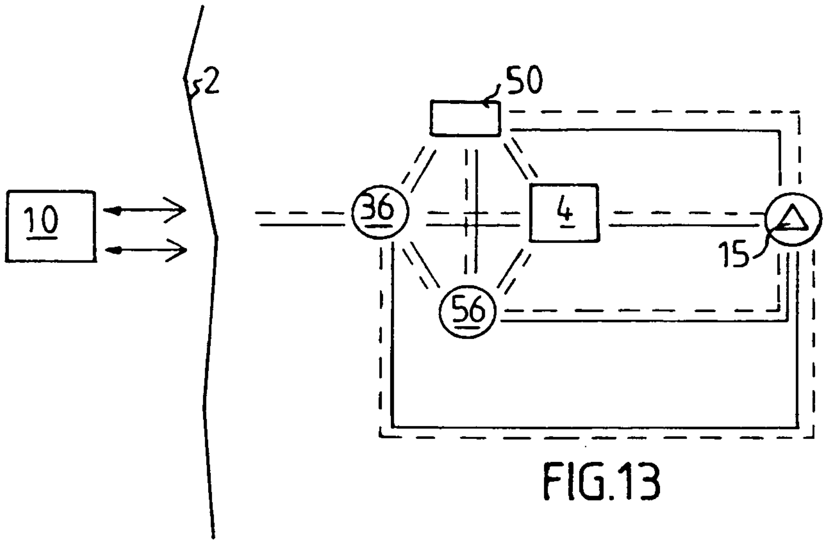


FIG.13

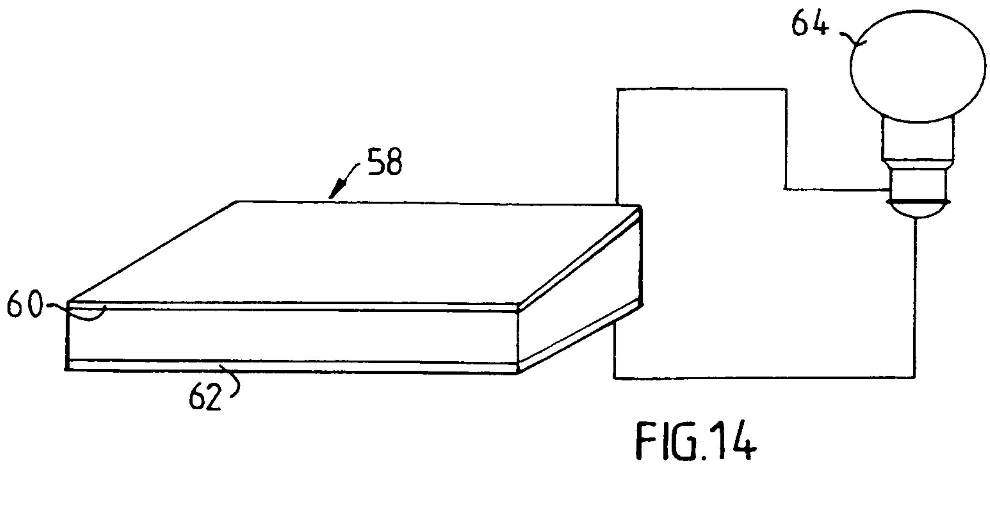


FIG.14

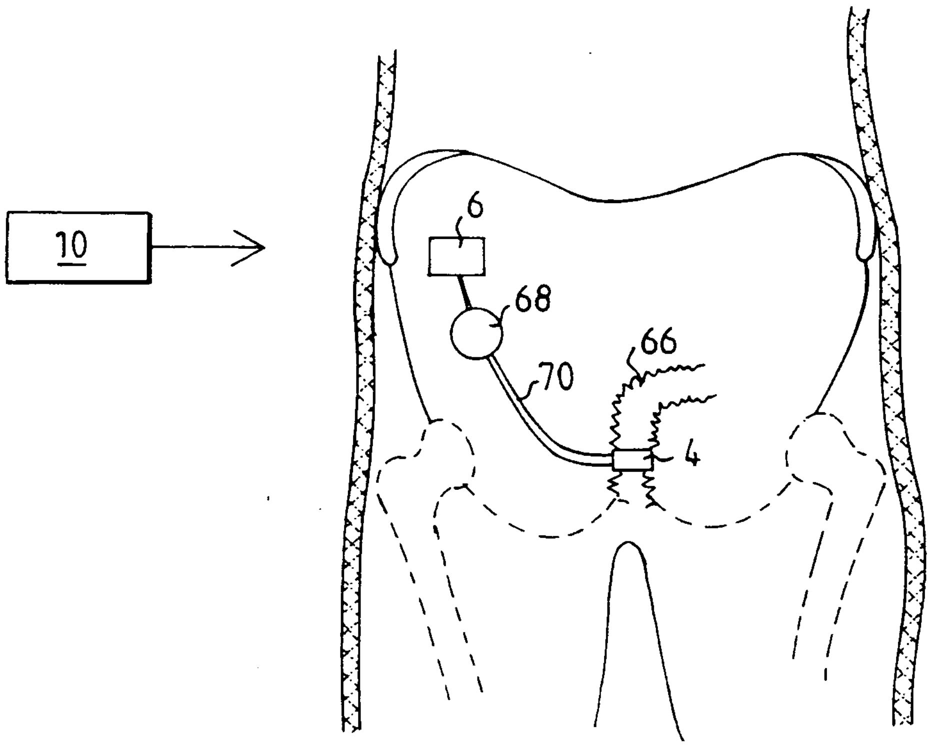


FIG.15

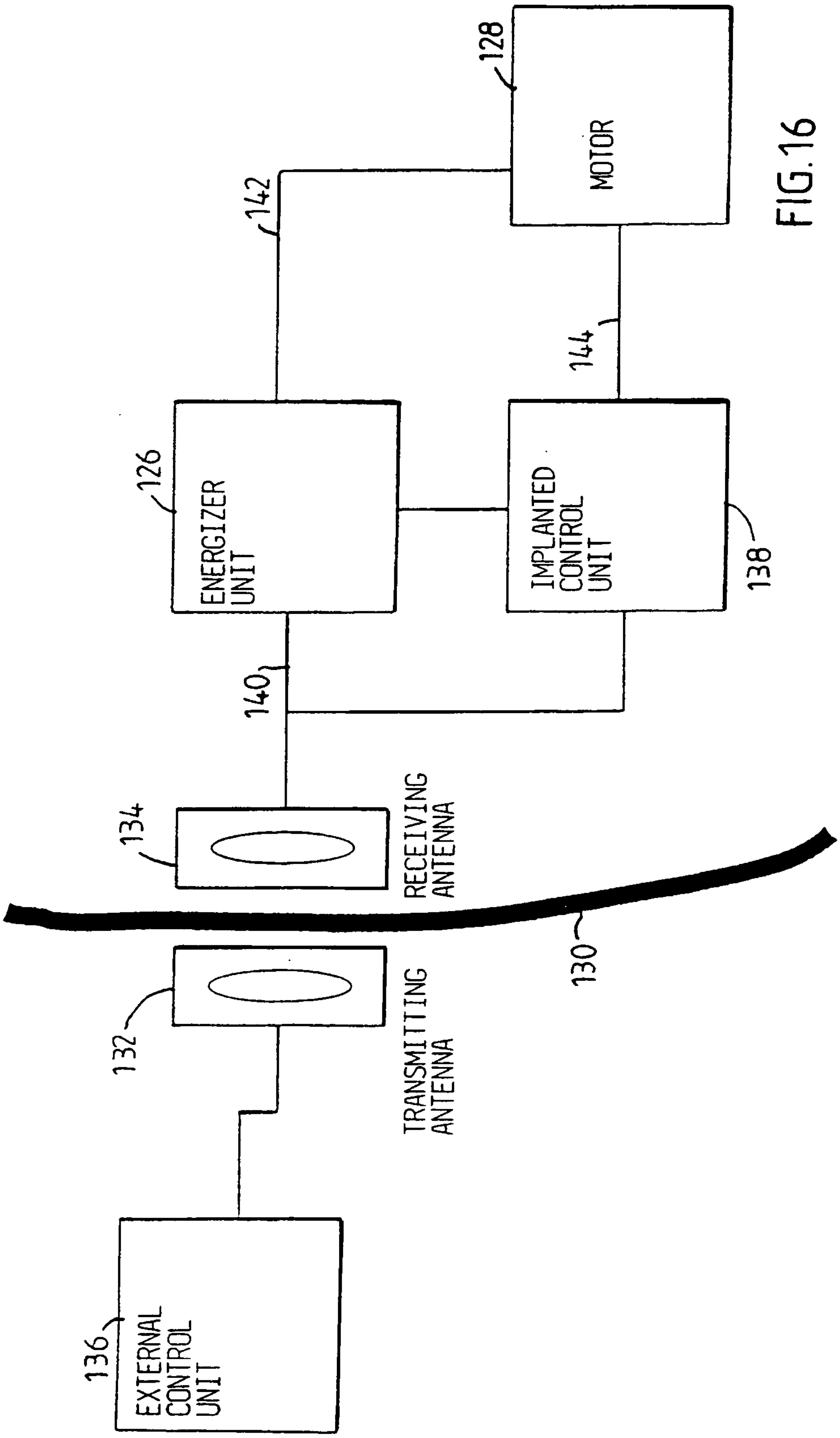
MOTOR
128
142
144
ENERGIZER UNIT
126
IMPLANTED CONTROL UNIT
138
140
RECEIVING ANTENNA
134
130
TRANSMITTING ANTENNA
132
EXTERNAL CONTROL UNIT
136

FIG.16

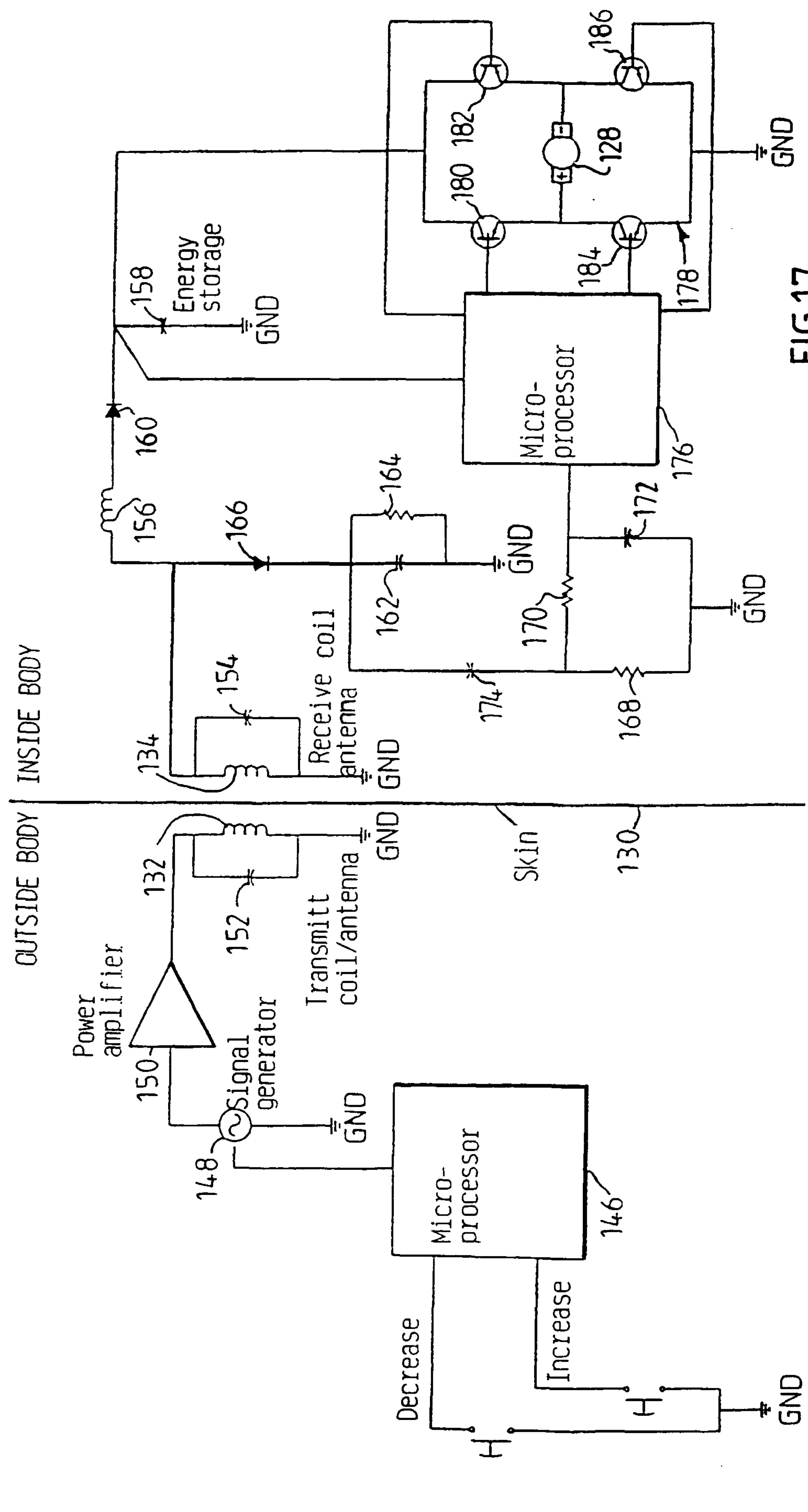
OUTSIDE BODY
INSIDE BODY
Power amplifier
150
Signal generator
148
GND
Micro-processor
146
Decrease
Increase
GND
132
Transmitt coil/antenna
152
GND
Skin
130
134
Receive coil antenna
154
GND
156
166
162
164
GND
174
168
170
172
GND
160
158
Energy storage
GND
Micro-processor
176
180
182
128
184
186
178
GND

FIG.17

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 5593443 A
- US 4222377 A
- FR 2692777